# EUROPÄISCHE PATENTSCHRIFT

(11) **EP 1 413 569 B1**
(45) Veröffentlichungstag und Bekanntmachung des Hinweises auf die Patenterteilung: **13.12.2006**
(21) Anmeldenummer: 03022855.5
(22) Anmeldetag: 08.10.2003
(51) Int. Cl.: C07C 69/608, C07D 307/93, C07D 311/94, C07D 335/04, A61K 6/00

(54) **Polymerisationsfähige bicyclische Cyclopropanderivate und deren Verwendung zur Herstellung von Dentalmaterialien**
Bicyclic cyclopropane derivatives capable of polymerisation and their use in the preparation of dental materials
Dérivés polymérisables du cyclopropane et leur utilisation dans la préparation de materiaux dentaires

(30) Priorität: 22.10.2002 DE 10249324
(43) Veröffentlichungstag der Anmeldung: 28.04.2004
(73) Patentinhaber: Ivoclar Vivadent AG, 9494 Schaan (LI)
(72) Erfinder: Moszner, Norbert, Prof. Dr., 9495 Triesen (LI); Zeuner, Frank, Dr., 9488 Schellenberg (LI); Fischer, Urs Karl, 9320 Arbon (CH); Meijere, Armin de, Prof. Dr., 37077 Göttingen (DE)
(74) Vertreter: UEXKÜLL & STOLBERG

(56) Entgegenhaltungen:
- EP-A- 0 943 601
- EP-A- 1 025 830
- MUELLER, LARRY G. ET AL: "Ring expansion synthesis of fused trans-.alpha.methylene-.gamma.-lactones" JOURNAL OF ORGANIC CHEMISTRY (1979), 44(25), 4741-2 , XP002269221
- MOSZNER, NORBERT ET AL: "Polymerization of cyclic monomers, 10: Synthesis and radical polymerization of methyl 2-(bicyclo[3.1.0]hex-1-yl)acrylate" MACROMOLECULAR RAPID COMMUNICATIONS (2003), 24(3), 269-273 , XP002269222
- FUMIO SANDA ET AL: "SYNTHESIS AND RADICAL POLYMERIZATION OF SPIROORTHOCARBONATES BEARING EXO-METHYLENE GROUPS" MACROMOLECULES, AMERICAN CHEMICAL SOCIETY. EASTON, US, Bd. 26, Nr. 4, 15. Februar 1993 (1993-02-15), Seiten 737-743, XP000360557 ISSN: 0024-9297
- FUMIO SANDA ET AL: "RADICAL POLYMERIZATION OF 3,9-DIMETHYLENE-1,5,7,11-TETRAOXASPIRO(5.5 ) UNDECANE. STUDY OF THE STRUCTURE OF THE POLYMER AND MECHANISM OF POLYMERIZATION" MACROMOLECULES, AMERICAN CHEMICAL SOCIETY. EASTON, US, Bd. 26, Nr. 4, 15. Februar 1993 (1993-02-15), Seiten 729-736, XP000360556 ISSN: 0024-9297
- MOSZNER N ET AL: "POLYMERIZATION OF CYCLIC MONOMERS" POLYMER BULLETIN, SPRINGER VERLAG. HEIDELBERG, DE, Bd. 40, Nr. 4/5, 1. April 1998 (1998-04-01), Seiten 447-453, XP000750410 ISSN: 0170-0839
- SANDA F ET AL: "RADICAL RING-OPENING POLYMERIZATION OF NOVEL VINYLCYCLOPROPANES DESIGNED AS LOW SHRINKAGE MONOMERS. STRUCTURE OF THE POLYMER, MECHANISM OF THE POLYMERIZATION, AND VOLUME CHANGE ON THE POLYMERIZATION" MACROMOLECULES, AMERICAN CHEMICAL SOCIETY. EASTON, US, Bd. 28, Nr. 5, 27. Februar 1995 (1995-02-27), Seiten 1346-1355, XP000492439 ISSN: 0024-9297

## Beschreibung

Die vorliegende Erfindung betrifft Zusammensetzungen, die polymersationsfähige bicyclische Cyclopropanderivate und einen Initiator für die radikalische Polymerisation enthalten, die sich insbesondere zur Herstellung von Dentalmaterialien eignen.

1,1-Disubstituierte 2-Vinylcyclopropane haben als radikalisch polymerisierbare Monomere zunehmendes Interesse gefunden, da bei der ringöffnenden Polymerisation im Vergleich zur Polymerisation von linearen Vinylmonomeren, wie z.B. Methacrylaten, eine geringere Volumenkontraktion stattfindet (N. Moszner, F. Zeuner, T. Völkel, V. Rheinberger, Macromol. Chem. Phys. 200 (1999) 2173).

Sanda et al. haben die Copolymerisation von 1,1-Bis(ethoxycarbonyl)-2-vinylcyclopropan (ECVCP) mit Methylmethacrylat (MMA) untersucht (F. Sanda, T. Takata, T. Endo, Macromolecules, 27 (1994) 3982). Die gefundenen Ergebnisse zeigen, daß sich ECVCP im Vergleich zu MMA durch ein geringeres radikalisches Polymerisationsvermögen auszeichnet, was seinen praktischen Einsatz deutlich einschränkt. Die Polymerisation von ECVCP und Methacrylaten führt zu heterogen zusammengesetzten Produkten mit einer geringen Einbindung des Vinylcyclopropans in die Copolymerstruktur, was unbefriedigende mechanische Eigenschaften zur Folge hat.

Aus der DE 198 12 888 A1 sind Vinylcyclopropanderivate und insbesondere Vinylcyclopropan(meth)acrylate bekannt, die sich gut mit Acrylaten und Methacrylaten copolymerisieren lassen.

Darüber hinaus sind Vinylcyclopropane mit mehreren polymerisationsfähigen Gruppen bekannt. F. Sanda, T. Takata, T. Endo, Macromolecules 27 (1994) 3986, beschreiben 1-Vinyl-5,7-dioxaspiro[2.5]octan-6-on, ein Hybridmonomer, das eine Vinylcyclopropan- und eine cyclische Carbonat-Gruppe enthält, und T. Okazaki, F. Sanda, T. Endo, Macromolecules 28 (1995) 6026, 1,10-Bis(vinyl)-4,8,12,15-tetraoxatrispiro[2.2.2.2.2.2]pentadecan, ein Monomer, bei dem zwei Vinylcyclopropangruppen über eine Spiroacetal-Einheit miteinander verbunden sind. Diese Verbindungen sind hydrolyseempfindlich und weisen im Vergleich zu monofunktionellen Vinylcyclopropanen keine verbesserte radikalische Copolymerisationsfähigkeit mit (Meth)acrylverbindungen auf.

Die DE 196 12 004 A1 offenbart multifunktionelle Vinylcyclopropanderivate mit zwei bis sechs Vinylcyclopropangruppen, die die Herstellung vernetzter Polymere erlauben.

Die EP 0 943 601 A1 offenbart Vinylcyclopropan-Derivate, die bei der radikalischen Polymerisation einen geringen Volumenschrumpf zeigen.

Der Erfindung liegt die Aufgabe zugrunde, Zusammensetzungen bereitzustellen die Monomere enthalten, die bei der radikalischen Polymerisation einen geringen Schrumpf zeigen und gleichzeitig eine mit Methacrylaten vergleichbare radikalische Polymerisationsfähigkeit aufweisen.

Diese Aufgabe wird durch Zusammensetzungen, die ein bicyclisches Cyclopropanderivat der allgemeinen Formel (I) enthalten gelöst. 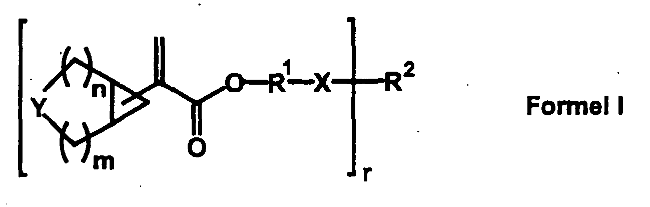 in der R¹, R², X, Y, n, m und r unabhängig voneinander die folgenden Bedeutungen haben:
n+m = 0 bis 8;
r = 1 bis 4;
R¹ = entfällt oder ein C₁-C₂₀-Alkylen-Rest, der durch O oder S unterbrochen sein kann, ein cycloaliphatischer C₄-C₁₂-Rest, ein bicyclischer C₄-C₁₂-Rest, ein C₆-C₁₄-Arylen- oder C₇-C₂₀-Alkylenarylen-Rest;
R² ist für r = 1: ein C₁-C₂₀-Alkyl-Rest, der durch O oder S unterbrochen sein kann, ein cycloaliphatischer C₄-C₁₂-Rest, ein bicyclischer C₄-C₁₂-Rest, ein C₆-C₁₄-Aryl- oder C₇-C₂₀-AlkylarylRest;
für r > 1: ein r-fach substituierter aliphatischer C₁- bis C₂₀-Rest, der durch O oder S unterbrochen sein kann, ein cycloaliphatischer C₄-C₁₂-Rest, ein aromatischer C₆-C₁₄-Rest oder aliphatisch-aromatischer C₇-C₂₀-Rest;
X = entfällt, -CO-O-, -CO-NH- oder -O-CO-NH- und
Y = CH₂, O oder S.

Für die Variablen der Formel (I) existieren die folgenden unabhängig voneinander wählbaren bevorzugten Definitionen:
n+m = 1 bis 5, insbesondere 2 oder 3;
r = 1 bis 3, insbesondere 1 oder 2;
R¹ = entfällt oder ein C₁-C₁₀-Alkylen-Rest, der durch O unterbrochen sein kann, Cyclohexylen, ein bicyclischer C₆-C₉-Rest, Phenylen oder ein C₇-C₁₀-Alkylenarylen-Rest, insbesondere entfällt, ein -(CH₂)₁₋₄- Rest, der durch ein O unterbrochen sein kann, Cyclohexylen oder Phenylen;
R² ist für r = 1: ein C₁-C₆-Alkyl-Rest, der durch O unterbrochen sein kann, ein cycloaliphatischer oder bicyclischer C₆-C₈-Rest, ein C₆-C₁₀-Aryl- oder C₇-C₁₀-Alkylaryl-Rest, insbesondere ein C₁-C₄-Alkyl-Rest, der durch ein O unterbrochen sein kann, Cyclohexyl, Bicyclo[2.2.1]heptyl;
für r > 1: ein r-fach substituierter aliphatischer C₁- bis C₁₂-Rest, der durch O unterbrochen sein kann, ein cycloaliphatischer C₅-C₇-Rest, ein aromatischer C₆-C₁₀-Rest oder aliphatisch-aromatischer C₇-C₁₀-Rest, insbesondere ein r-fach substituierter aliphatischer C₂- bis C₆-Rest, ein r-wertiger Cyclohexan-Rest oder ein r-wertiger Benzol-Rest;
X = entfällt, -CO-O- oder -O-CO-NH-, insbesondere entfällt oder -CO-O- und
Y = CH₂ oder O, insbesondere CH₂.

Naturgemäß sind solche bicyclischen Cyclopropanderivate besonders bevorzugt, bei denen alle Variablen eine der bevorzugten oder besonders bevorzugten Bedeutungen aufweisen.

Die Angabe, daß ein Rest durch Fremdatome, wie Sauerstoff oder Schwefel, unterbrochen sein kann, ist so zu verstehen, daß eines oder mehrere der Fremdatome in eine Kohlenstoffkette integriert sind. Daraus folgt, daß die Fremdatome nicht endständig sein können, d.h. eine Anbindung an Nachbargruppen immer über ein Kohlenstoffatom erfolgt, und daß die Zahl der Fremdatome zwangsläufig kleiner als die Zahl der Kohlenstoffatome sein muß. Bevorzugt sind Gruppen die maximal 4, vorzugsweise maximal 2 Fremdatome aufweisen. Das Gesagte gilt sinngemäß auch für verzweigte Gruppen.

Durch die Formel (I) werden sämtliche stellungs- und stereoisomeren Formen sowie Gemische verschiedener stellungs- und stereoisomerer Formen, wie z.B. Racemate, erfaßt. Wie Formel (I) zu entnehmen ist, kann der Rest -C(=CH₂)-C(=O)-O-R¹-X-R² über ein Brückenkopfatom oder das Brückenatom an den Cyclopropanring gebunden sein.

Die Reste R¹ und R² können ein- oder mehrfach substituiert und unsubstituiert sein. Bevorzugte Substituenten für R¹ sind Alkyl, Halogen, OCH₃, OC₂H₅, Vinyl, Propenyl, (Meth) acryl, COOR³ und mesogene Gruppen.

Bevorzugte Substituenten für R² sind für r = 1 Alkyl, Halogen, OCH₃, OC₂H₅, Vinyl, Propenyl, (Meth) acryl, COOR³, SiCl₃, Si(OR⁴)₃, oder mesogene Gruppen. Bevorzugte Substituenten für r > 1 sind Alkyl, Halogen, OCH₃, OC₂H₅, Vinyl, Propenyl, (Meth)acryl, CO-OR³ und mesogene Gruppen.

Wenn nicht anders angegeben steht Alkyl vorzugsweise für C₁- bis C₁₀-Alkyl, insbesondere C₁- bis C₄-Alkyl. Bevorzugte Halogensubstituenten sind F, Cl, Br und I.

R³ hat in allen Fällen die Bedeutung H, C₁- bis Cio- Alkyl-, insbesondere C₁- bis C₄-Alkyl, oder Phenyl, und R⁴ die Bedeutung H oder C₁- bis C₁₀-Alkyl, insbesondere C₁- bis C₄-Alkyl.

Unter mesogenen Gruppen werden solche Reste verstanden, die sogenannte Mesophasen, d.h. flüssigkristalline Phasen bilden können. Bevorzugte mesogenen Gruppen sind:

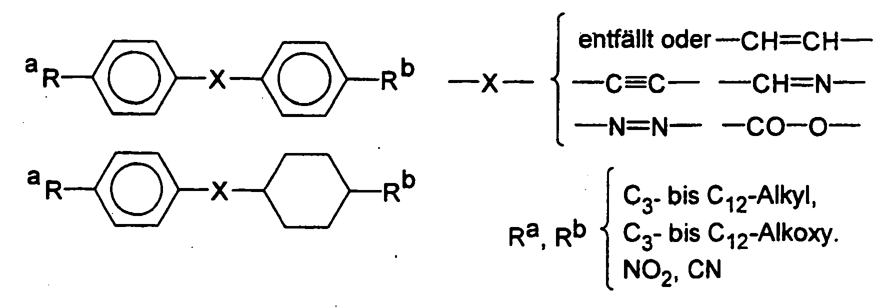

Bevorzugt sind bicyclische Cyclopropanderivate bei denen die Reste einfach substituiert oder unsubstituiert sind.

Die bicyclischen Cyclopropanderivate der erfindungsgemässen Zusammensetzung der allgemeinen Formel (I) (R¹, X entfällt, r=1) können ausgehend von Cycloalkencarbaldehyden erhalten werden:

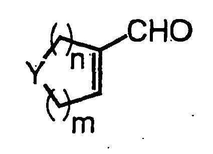

Diese können aus Cycloalkenen via 1,2-Dihydroxycycloalkane durch stufenweise Oxidation hergestellt werden (L.F. Tietze, T. Eicher, *Reaktionen und Synthesen* Thieme Verlag Stuttgart, 1991 S. 62 und 266). Durch Addition von Blausäure an die Cycloalkencarbaldehyde und nachfolgende Alkoholyse erhält man 2-(Cycloalken-1-yl)-2-hydroxyessigsäureester (E.G.J.C. Warmerdam, A.M.C.H. van den Nieuwendijk, C.G. Kruse, J. Brussee, A. van der Gen, Rec. Trav. Chim. Pays-Bas 115 (1996) 20) :

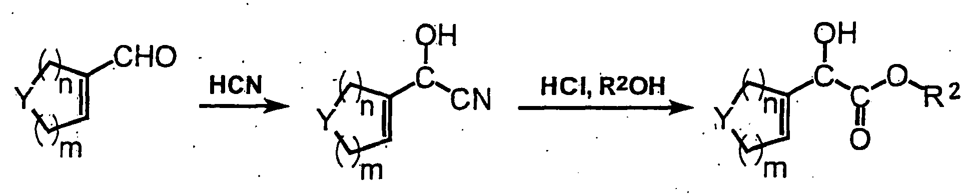

Im nächsten Schritt findet die Cyclopropanierung nach Simmons-Smith statt (Zh. Yang, J.C. Lorenz, Y. Shi, Tetrahedron Lett: 39 (1998) 8621) :

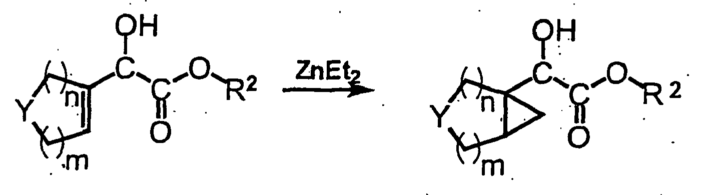

Die Bicyclo-2-hydroxyessigsäureester können anschließend z.B. durch Oxidation mit MnO₂ in die Bicyclo-2-oxo-essigsäureester überführt werden (N.G. Capps, G.M. Davies, D. Loakes, R.W. McCabe, D.W. Young, J. Chem. Soc. Perkin Trans. 1 (1991) 3077). Durch eine Wittig-Reaktion gelangt man schließlich zu den bicyclischen Cyclopropanderivaten der allgemeinen Formel (I):

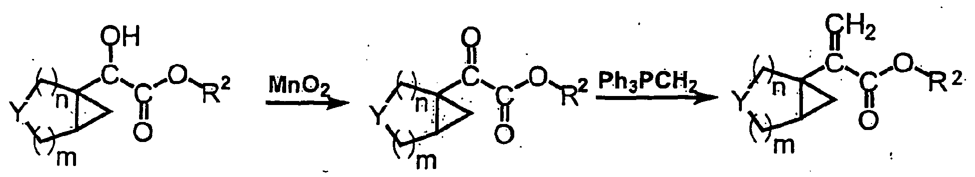

### Konkretes Beispiel:

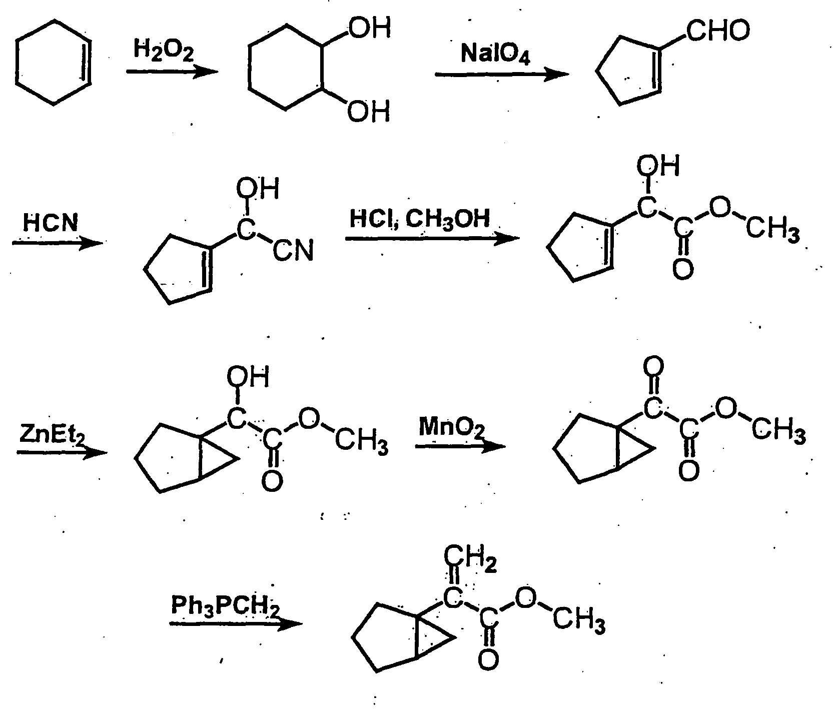

Bicyclische Cyclopropanderivate der allgemeinen Formel (I) (r >1, R² entfällt) können durch Hydrolyse von bicyclischen Cyclopropanderivaten (r=1 und R¹, X = entfällt) und nachfolgende Veresterung mit polyfunktionellen Alkoholen [(HO-R¹-X-)ᵣ] erhalten werden:

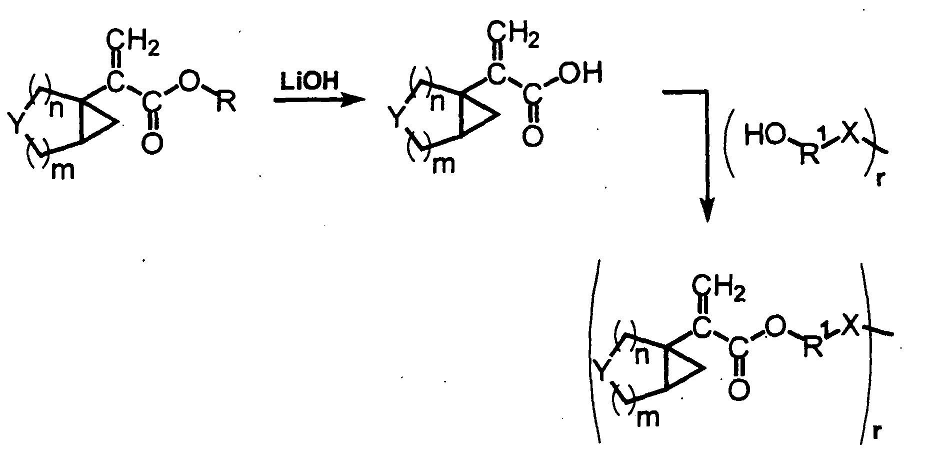

### Konkretes Beispiel:

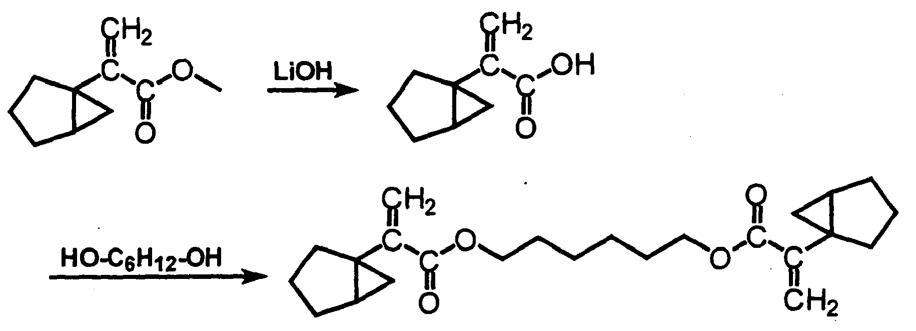

Besonders bevorzugte Beispiele für die bicyclischen Cyclopropanderivate der Formel (I) der erfindungsgemässen Zusammensetzungs sind:

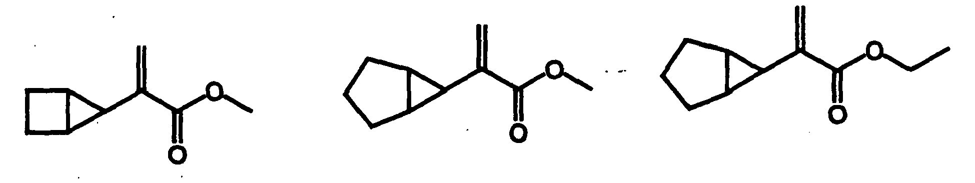

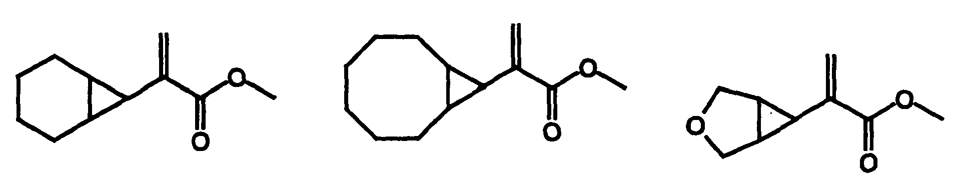

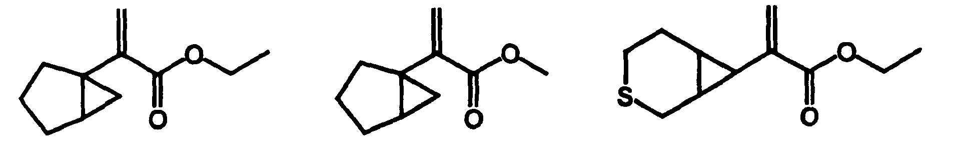

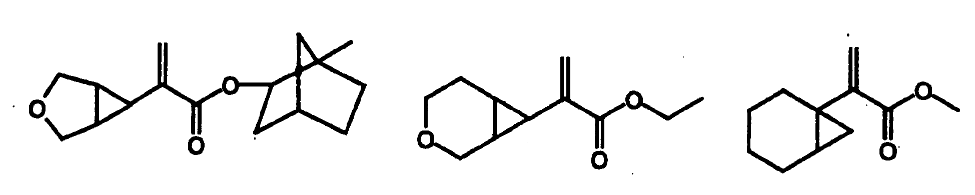

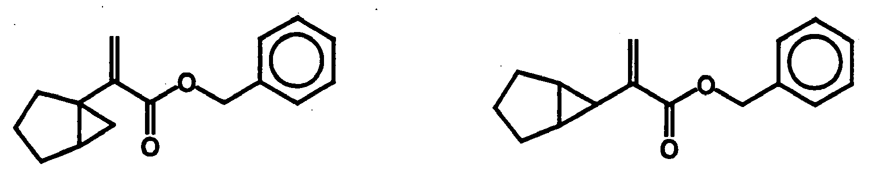

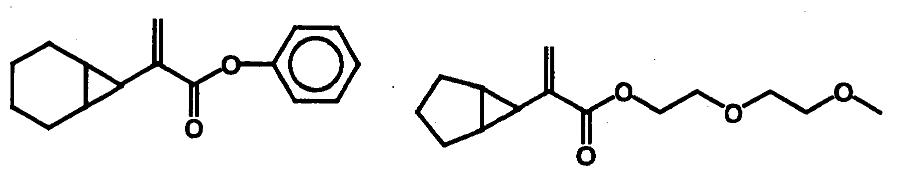

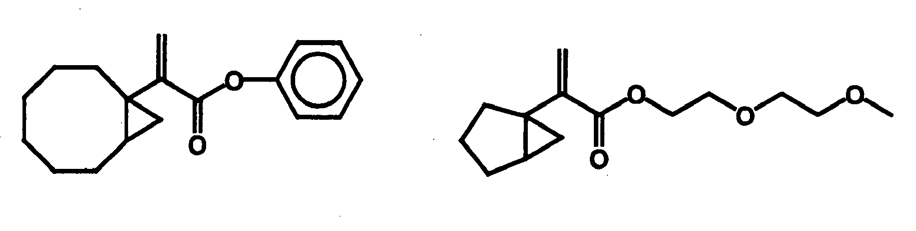

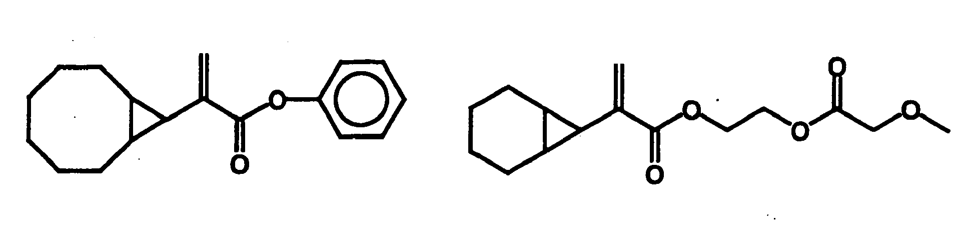

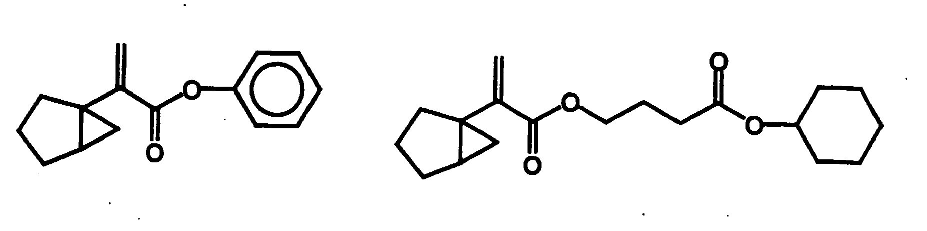

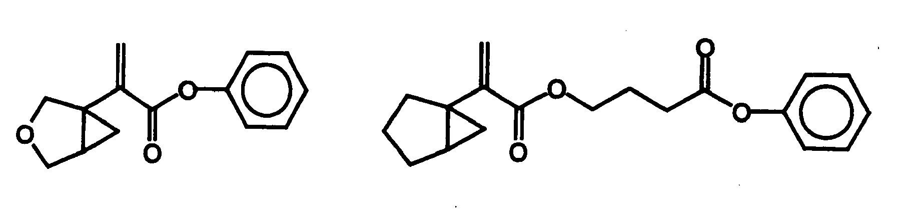

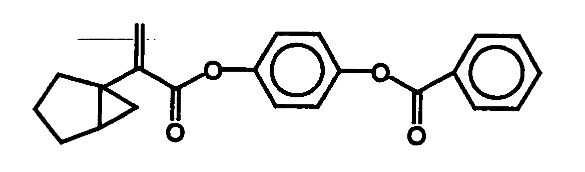

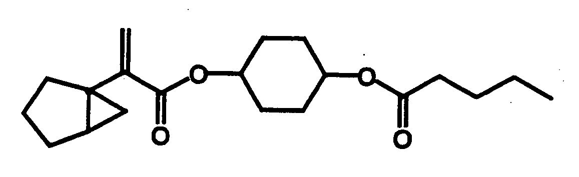

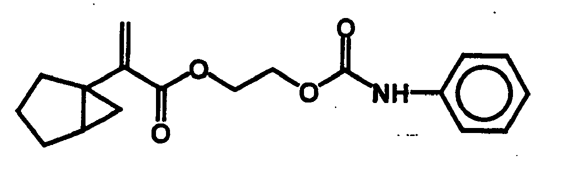

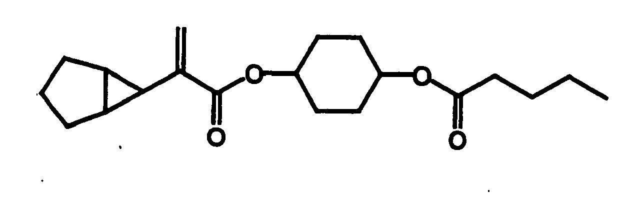

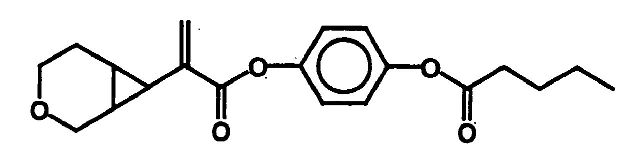

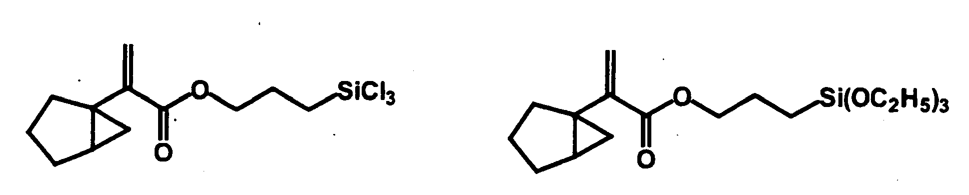

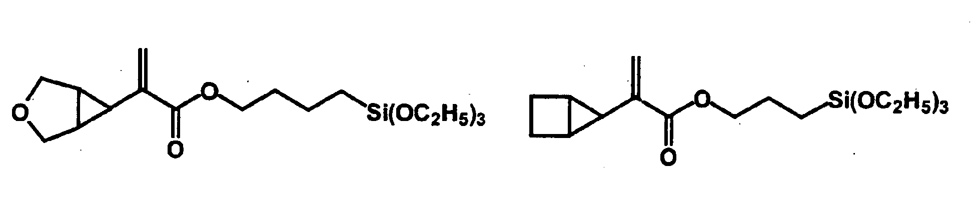

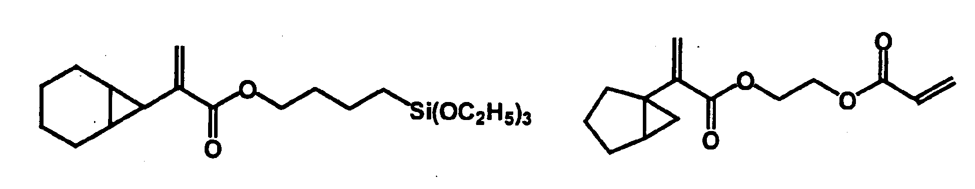

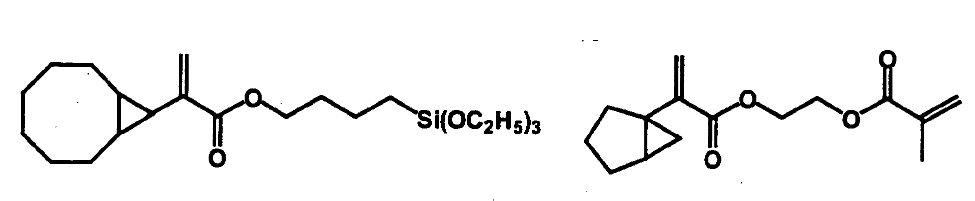

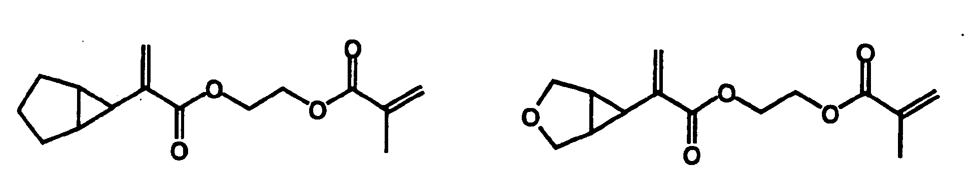

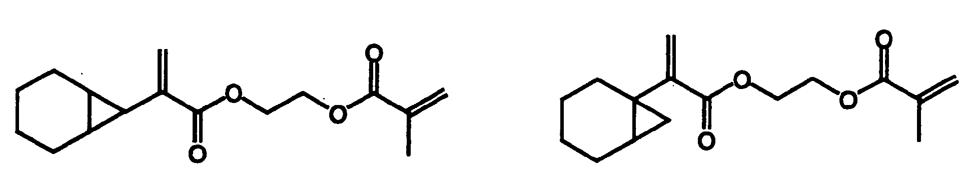

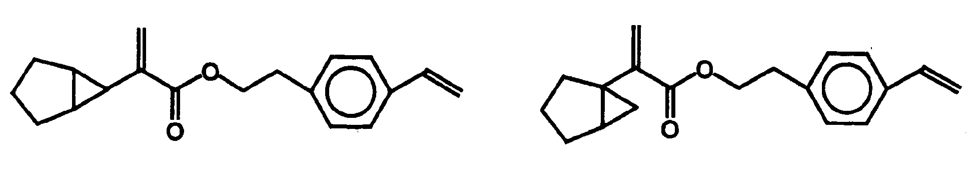

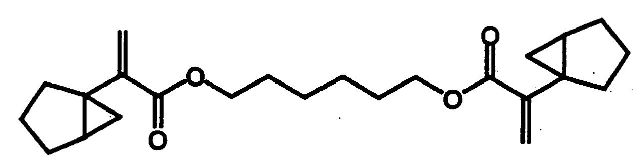

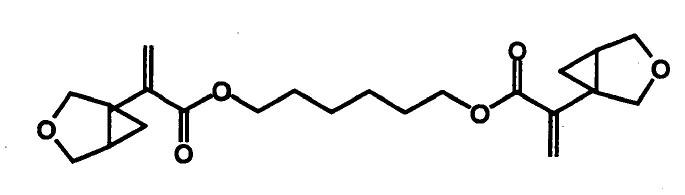

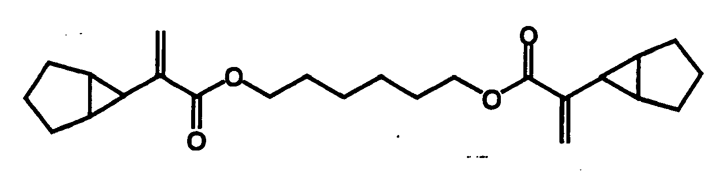

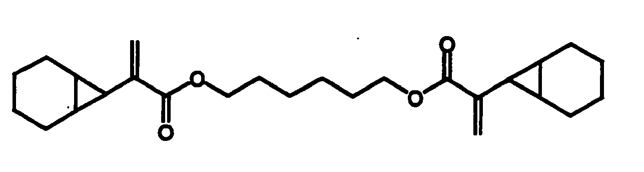

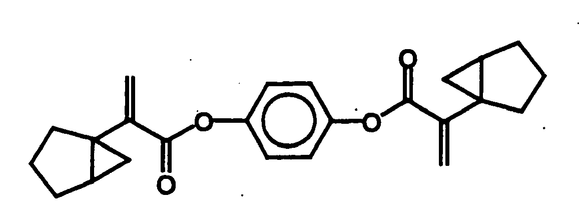

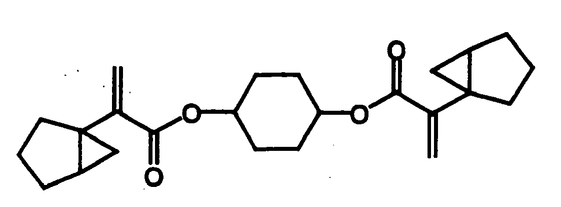

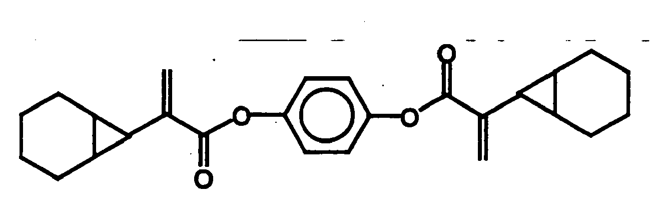

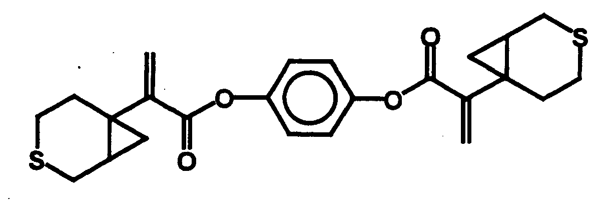

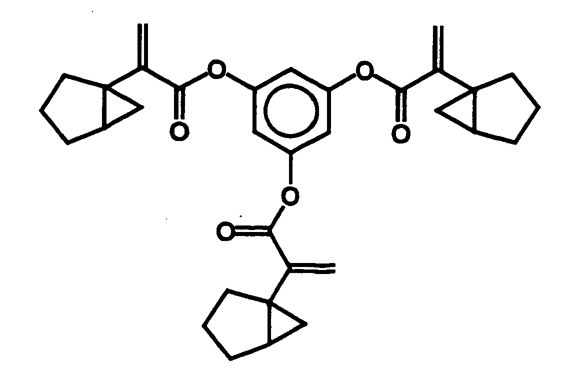

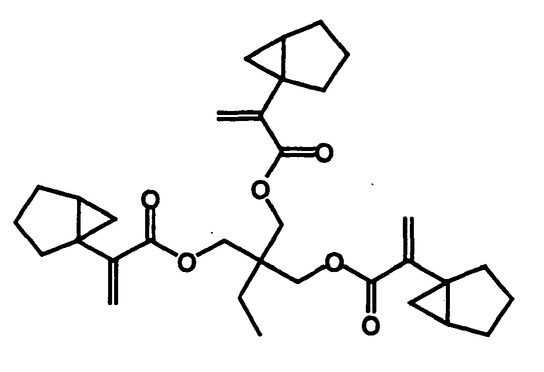

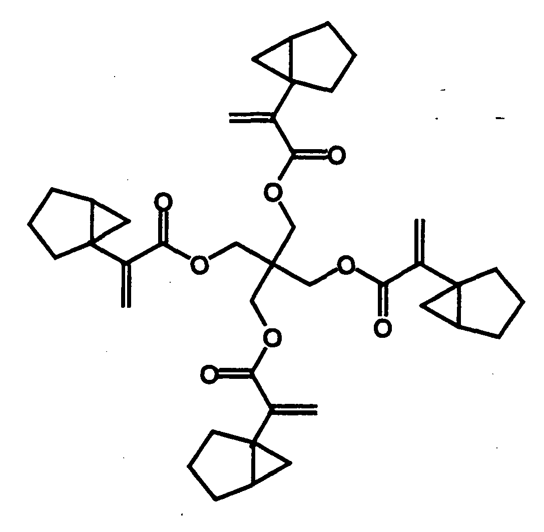

Die erfindungsgemäßen Zusammensetzungen eignen sich besonders zur Herstellung von Polymeren und Copolymeren, Formkörpern, Adhäsiven, Beschichtungsmaterialien, Zementen und Kompositen und insbesondere Dentalmaterialien.

Hierzu werden sie vorzugsweise mit zusätzlichen Monomeren, Füllstoffen und ggf. weiteren Hilfsstoffen gemischt. Die so erhaltenen Zusammensetzungen können durch radikalische Polymerisation gehärtet werden. Sowohl die gehärteten Produkte, wie z.B. Polymere und Formkörper, als auch die härtbaren Zusammensetzungen sind ebenfalls Gegenstand der Erfindung.

Als Initiatoren für die radikalische Polymerisation eignen sich die bekannten Initiatoren für die Kalt-, Heiß- und Photohärtung. Geeignete Initiatoren werden beispielsweise in der Encyclopedia of Polymer Science and Engineering, Vol. 13, Wiley-Intersci. Pub., New York etc. 1988, S. 754 ff. beschrieben.

Bevorzugte Initiatoren sind Azoverbindungen, wie Azobis(isobutyronitril) (AIBN) oder Azobis(4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich besonders Benzpinakol und 2,2'-Di(C₁-C₈-alkyl)benzpinakole.

Geeignete Photoinitiatoren für den UV- oder sichtbaren Bereich werden von J.P. Fouassier, J.F. Rabek (Hrsg.), Radiation Curing in Polymer Science and Technology, Vol. II, Elsevier Applied Science, London und New York 1993, Seiten 155 bis 237, beschrieben. Bevorzugte Photoinitiatoren sind Benzoinether, Dialkylbenzilketale, Dialkoxyacetophenone, Acylphosphinoxide, Bisacylphosphinoxide, α-Diketone, wie 10-Phenanthrenchinon, Diacetyl, Furil, Anisil, 4,4'-Dichlorbenzil und 4,4'-Dialkoxybenzil und Kampferchinon.

Zur Herstellung von Dentalmaterialien sind Dibenzoylperoxid, Campferchinon und Acylphosphinoxide bevorzugt.

Zur Beschleunigung der Initiierung durch Peroxide oder α-Diketone eignen sich besonders Kombinationen mit aromatischen Aminen. Als Beschleuniger sind zudem Redoxsysteme einsetzbar, insbesondere Kombinationen aus Benzoylperoxid, Lauroylperoxid oder Campherchinon mit Aminen, wie N,N-Dimethyl-p-toluidin, N,N-Dihydroxyethyl-p-toluidin, p-Dimethylaminobenzoesäureethylester oder strukturverwandten Aminen. Darüber hinaus sind auch Redoxsysteme geeignet, die neben Peroxid Ascorbinsäure, ein Barbiturat oder eine Sulfinsäure als Reduktionsmittel enthalten.

Die bicyclischen Cyclopropanderivate der erfindungsgemässen Zussammensetzung lassen sich allein oder in Mischung mit herkömmlichen radikalisch polymerisierbaren Monomeren polymerisieren. Zusammensetzungen, die neben einem oder mehreren bicyclischen Cyclopropanderivaten und Initiator zusätzlich ein oder mehrere radikalisch polymerisierbare Monomere enthalten, sind bevorzugt.

Als Comonomere eignen sich monofunktionelle und/oder mehrfunktionelle radikalisch polymerisierbare Monomere, insbesondere di-, tri- und tetrafunktionelle, ganz besonders bevorzugt difunktionelle Vernetzermonomere. Unter monofunktionellen Monomeren werden Verbindungen mit einer, unter mehrfunktionellen Monomeren Verbindungen mit zwei und mehr radikalisch polymerisierbaren Gruppen verstanden. Für die Herstellung von Adhäsiven, Beschichtungsmaterialien und Dentalmaterialien eignen sich vor allem vernetzende bi- oder mehrfunktionelle Acrylate oder Methacrylate, wie z.B. Bisphenol-A-di(meth)acrylat, Bis-GMA (das Additionsprodukt von Methacrylsäure und Bisphenol-A-diglycidylether), UDMA (das Additionsprodukt von Hydroxyethylmethacrylat und 2,2,4-Trimethylhexamethylendiisocyanat), Di-, Tri- oder Tetraethylenglykol-di(meth)acrylat, Decandioldi(meth)acrylat, Trimethylolpropan-tri(meth)acrylat und Pentaerythrittetra(meth)acrylat. Es eignen sich ebenfalls die durch Veresterung von (Meth)acrylsäure mit den entsprechenden Diolen zugänglichen Verbindungen Butandiol-di(meth)acrylat, 1,10-Decandioldi(meth)acrylat und 1,12-Dodecandioldi(meth)acrylat, sowie di- und mehrfunktionelle 2-Vinylcyclopropanderivate, die durch Umsetzung von 1-Methoxycarbonyl-2-vinylcyclopropan-1-carbonsäure mit zwei- oder mehrwertigen OH- oder NH₂-Verbindungen als Kopplungskomponente, d.h. z.B. mit Ethylenglycol, Di- oder Triethylenglycol, Butylenglycol, 1,6-Hexandiol, Glycerin, Triethanolamin, Trimethylolpropantriol, Pentaerythrit oder Glucose, sowie Hydrochinon, Resorcin, Brenzkatechin oder Pyrogallol, Ethylendiamin, Propylendiamin, Hexamethylendiamin, o-, p- oder m-Phenylendiamin, zugänglich sind.

Weitere mehrfunktionelle radikalisch polymerisierbar Monomere, die sich besonders als Vernetzermonomere eignen, sind Urethane aus 2-(Hydroxymethyl)acrylsäureethylester und Diisocyanten, wie z.B. 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder N,N'-Bis(acryloyl)-piperazin, die durch Umsetzung aus den entsprechenden Diaminen mit (Meth)acrylsäurechlorid synthetisiert werden können. Diese Verbindungen zeichnen sich durch eine relativ hohe Hydrolysestabilität aus.

Zudem eignen sich die in der DE 101 01 523 und DE 102 28 540 offenbarten Acrylamide und Hydroxyalkylacrylamide als radikalisch polymerisierbare Comonomere.

Bevorzugte monofunktionelle radikalisch polymerisierbare Monomere, die sich besonders als Verdünnermonomere eignen, sind hydrolysestabile Mono(meth)acrylate, wie z.B. Mesitylmethacrylat, oder 2-(Alkoxymethyl)acrylsäuren, wie z.B. 2-(Ethoxymethyl)acrylsäure, 2-(Hydroxymethyl)acrylsäure, N-mono- oder -disubstituierte Acrylamide, wie z.B. N-Ethylacrylamid, N,N-Dimethacrylamid, N-(2-Hydroxyethyl)acrylamid oder N-(2-Hydroxyethyl)-N-methyl-acrylamid, sowie N-monosubstituierte Methacrylamide, wie z.B. N-Ethylmethacrylamid oder N-(2-Hydroxyethyl)methacrylamid und außerdem N-Vinylpyrrolidon oder Allylether.

Als Füllstoffe zur Verbesserung der mechanischen Eigenschaften oder zur Einstellung der Viskosität eignen sich alle organischen und anorganischen Partikel oder Fasern.

Bevorzugte Füllstoffe zur Herstellung von Dentalmaterialien wie Befestigungszemente oder Füllungskomposite sind amorphe, kugelförmige Materialien auf der Basis von Oxiden, wie ZrO₂ und TiO₂, bzw. Mischoxiden aus SiO₂, ZrO₂ und/oder TiO₂ mit einer mittleren durchschnittlichen Partikelgröße von 0,005 bis 2,0 µm, vorzugsweise von 0,1 bis 1 µm, wie sie beispielsweise in der DE-PS 32 47 800 offenbart werden, nanopartikuläre oder mikrofeine Füllstoffe, wie pyrogene Kieselsäure oder Fällungskieselsäure, sowie Mini-Füllstoffe, wie Quarz-, Glaskeramik- oder Glaspulver, sowie röntgenopake Füllstoffe, wie Ytterbiumfluorid oder nanopartikuläres Tantal(V)oxid oder Bariumsulfat. Unter Mini-Füllstoffen werden Füllstoffe mit einer durchschnittlichen Partikelgröße von 0,5 bis 1,5 µm, vorzugsweise 0,01 bis 1 µm verstanden. Außerdem können auch Glasfasern, Polyamid- oder Kohlenstoff-Fasern eingesetzt werden.

Darüber hinaus können die erfindungsgemäßen Zusammensetzungen im Bedarfsfall weitere Hilfsstoffe enthalten, insbesondere Stabilisatoren, UV-Absorber, Farbstoffe, Pigmente und/oder Gleitmittel. Unter Stabilisatoren werden solche Stoffe verstanden, die eine vorzeitige Polymerisation verhindern und damit vor allem die Lagerstabilität von Monomermischungen und Kompositen erhöhen, ohne jedoch die Eigenschaften der ausgehärteten Materialien zu beeinträchtigen. Bevorzugte Stabilisatoren sind Hydrochinonmonomethylether (MEHQ) und 2,6-Di-tert.-butyl-4-methylphenol (BHT).

Die erfindungsgemäßen Zusammensetzungen eignen sich besonders als Dentalmaterialien, insbesondere als Dentaladhäsive, Befestigungszemente oder Füllungskomposite sowie Materialien für Inlays/Onlays, Zähne oder Verblendmaterialen für Kronen und Brücken. Sie zeichnen sich durch einen geringen Polymerisationschrumpf und, nach der Härtung, sehr gute mechanische Eigenschaften aus.

Zur Verwendung als Dentalmaterialien sind härtbare Zusammensetzungen bevorzugt, die
a) 1 bis 95 Gew.-% bicyclisches Cyclopropanderivat;
b) 0,01 bis 5 Gew.-% Initiator für die radikalische Polymerisation; und
c) 0 bis 94 Gew.-% weiteres radikalisch polymerisierbares Monomer;
enthalten.

Zur Verwendung als Adhäsiv und insbesondere dentales Adhäsiv eignen sich besonders Zusammensetzungen, die
a) 1 bis 80 Gew.-%, und besonders bevorzugt 10 bis 60 Gew.-% erfindungsgemäßes bicyclisches Cyclopropanderivat,
b) 0,01 bis 5 Gew.-%, besonders bevorzugt 0,1 bis 2,0 Gew.-% Initiator für die radikalische Polymerisation,
c) 0 bis 60 Gew.-% und besonders bevorzugt 0 bis 40 Gew.-% weiteres radikalisch polymerisierbares Monomer,
d) 0 bis 20 Gew.-% Füllstoff und
e) 0 bis 40 Gew.-% und besonders bevorzugt 0 bis 30 Gew.-% Lösungsmittel
enthalten

Zur Verwendung als Zement und insbesondere dentaler Zement eignen sich besonders Zusammensetzungen, die
a) 1 bis 60 Gew.-%, und besonders bevorzugt 20 bis 50 Gew.-% erfindungsgemäßes bicyclisches Cyclopropanderivat,
b) 0,01 bis 5 Gew.-%, besonders bevorzugt 0,1 bis 2,0 Gew.-% Initiator für die radikalische Polymerisation,
c) 0 bis 60 Gew.-% und besonders bevorzugt 0 bis 20 Gew.-% weiteres radikalisch polymerisierbares Monomer,
d) 20 bis 60 Gew.-% und besonders bevorzugt 30 bis 60 Gew.-% Füllstoff
enthalten.

Zur Verwendung als Füllungskomposit und insbesondere dentaler Füllungskomposit eignen sich besonders Zusammensetzungen, die
a) 1 bis 45 Gew.-%, und besonders bevorzugt 10 bis 30 Gew.-% erfindungsgemäßes bicyclisches Cyclopropanderivat,
b) 0,01 bis 5 Gew.-%, besonders bevorzugt 0,1 bis 2,0 Gew.-% Initiator für die radikalische Polymerisation,
c) 0 bis 50 Gew.-% und besonders bevorzugt 0 bis 10 Gew.-% weiteres radikalisch polymerisierbares Monomer,
d) 30 bis 85 Gew.-% und besonders bevorzugt 40 bis 80 Gew.-% Füllstoff
   enthalten.

Zur Verwendung als Beschichtungsmaterial und insbesondere dentales Beschichtungsmaterial eignen sich besonders Zusammensetzungen, die
a) 1 bis 95 Gew.-%, und besonders bevorzugt 10 bis 60 Gew.-% erfindungsgemäßes bicyclisches Cyclopropanderivat,
b) 0,01 bis 5 Gew.-%, besonders bevorzugt 0,1 bis 2,0 Gew.-% Initiator für die radikalische Polymerisation,
c) 0 bis 60 Gew.-% und besonders bevorzugt 0 bis 40 Gew.-% weiteres radikalisch polymerisierbares Monomer,
d) 0 bis 20 Gew.-% Füllstoff
enthalten.

Im Folgenden wird die Erfindung anhand von Ausführungsbeispielen näher erläutert.

### Beispiele

### Beispiel 1:

### 1. Stufe: 2-(Bicyclo[3.1.0]hex-1-yl)-2-hydroxyessigsäuremethylester

Zu einer gut gerührten Lösung von 31,8 ml Diethylzink (310 mmol) in 400 ml wasserfreiem Methylenchlorid wurden bei -15 °C innerhalb 30 Min 23,8 ml (310 mmol) Trifluoressigsäure, gelöst in 25 ml Methylenchlorid, so zugetropft, daß die Temperatur 0°C nicht überschritt. Nach weiteren 15 min Rühren bei 0°C wurden 25,1 ml (310 mmol) Diiodmethan so zugesetzt, daß die Temperatur 0°C nicht überschritt. Die Mischung wurde gerührt bis sie vollständig homogen war und dann bei 0°C mit 22,0 g (141 mmol) 2-(Cyclopenten-1-yl)-2-oxyessigsäuremethylester versetzt. Die Mischung wurde für weitere 12 Stunden bei Raumtemperatur gerührt, dann auf 0°C gekühlt und vorsichtig unter starkem Rühren mit 310 ml 1M Schwefelsäure versetzt. Die wäßrige Phase wurde mit Kochsalz gesättigt und danach mit Diethylether extrahiert. Die vereinigten organischen Phasen wurden mit 2N Natronlauge und gesättigter Kochsalzlösung gewaschen und über Magnesiumsulfat getrocknet. Die Lösung wurde eingeengt und der Rückstand im Vakuum destilliert. Es wurden 22,8 g (95 % Ausbeute einer farblosen Flüssigkeit mit einem Siedepunkt von Kp_{0.1Torr} = 53-54°C erhalten.

IR (film): ν = 3490, 3067, 3000, 2953, 2862, 1735 (C=O), 1438, 1273, 1226, 1201, 1085, 1026, 990, 946, 798, 776, 730.
¹H-NMR (250 MHz, CDCl₃): δ = 0:43-0.55 (m, 2 H), 1.08-1.30 (m, 1 H), 1.36-1.81 (m, 6 H), 2.87 (br. s, 1 H), 3.79 (s, 3 H), 3.99 (s, 1 H).
¹³C-NMR (62.9 MHz, CDCl₃, DEPT): δ = 9.9 (-), 21.1 (-), 22.0 (+), 26.8 (-), 28.3 (-), 31.6 (q), 52.4 (+), 74.3 (+), 175.0 (q).
MS (70 eV, EI), m/z (%): 170.1 (0.8) [M⁺], 152 (25.4), 137 (5.4), 120 (4.8), 111 (67.5), 93 (25.2), 81 (100.0), 67 (67.5).- C₉H₁₄O₃ (170.21) : Ber. C 63.51, H 8.29; Gef. C 63.28, H 7.99.

### 2. Stufe: 2-(Bicyclo[3.1.0]hex-1-yl)-2-oxoessigsäuremethylester

Zu einer gut gerührten Lösung von 13.6 g (80 mmol) 2-(Bicyclo[3.1.0]hex-1-yl)-2-hydroxyessigsäuremethylester in 500 ml wasserfreiem Äther wurden 2 × 40 g aktives Mangan(IV)-oxid in 2 Stunden nacheinander zugegeben und die Mischung bei Raumtemperatur für weitere 4 Stunden gerührt, bis das gesamte Ausgangsmaterial verbraucht war (DC-Kontrolle). Dann wurde das Reaktionsgemisch über Kieselgur (Celite®) filtriert. Nach dem Abdampfen des Lösungsmittels im Vakuum bei Raumtemperatur erhielt man 10.8 g (80%) an 2-(Bicyclo[3.1.0]hex-1-yl)-2-oxoessigsäuremethylester als klare schwach-gelbe Flüssigkeit (Reinheit >96% nach GC), die nicht weiter gereinigt werden muss.

IR (Film): ν = 3008, 2957, 2869, 1734 (C=O), 1684 (C=O), 1453, 1437, 1382, 1293, 1240, 1179, 1081, 1039, 1003, 929, 866, 794. ¹H-NMR (250 MHz, CDCl₃) : δ = 1.14, (t, ***J*** = 5.50 Hz, 1 H), 1.18-1.36 (m, 1 H), 1.60-1.94 (m, 5 H), 2.08-2.22 (m, 1 H), 2.31-2.38 (m, 1 H), 3.79 (s, 3 H).
¹³C-NMR (62.9 MHz, CDCl₃, DEPT): δ = 19.0 (_), 20.4 (_), 26.5 (_), 26.9 (-), 33.9 (+), 39.3 (q), 52.3 (+), 163.3 (q), 195.8 (q).
MS (70 eV, EI), m/z (%): 168 (0.6) [M⁺], 140 (1.6), 109 (100.0), 81 (81.3), 79 (41.3).
- C₉H₁₂O₃ (168.19): Ber. C 64.27, H 7.19; Gef. C 64.55, H 6.96.

### 3. Stufe: 2-(Bicyclo[3.1.0]hex-1-yl)acrylsäuremethylester (BCHAM)

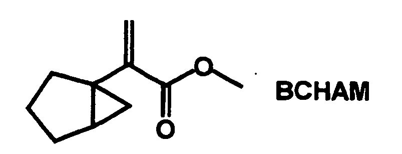

Zu einer gut gerührten Suspension von 37,5 g (105 mmol) Methyltriphenylphosphoniumbromid in 400 ml wasserfreiem Tetrahydrofuran (THF) wurden unter Argon bei -78 °C 1,4 ml (10 mmol) Diisopropylamin sowie 65 ml (100 mmol) 1,55 M n-BuLi-Lösung in Hexan zugesetzt. Das Reaktionsgemisch wurde auf Raumtemperatur erwärmen gelassen, wieder gekühlt und dann bei - 78 °C eine Lösung von 16,8 g (100 mmol) 2-(Bicyclo-[3.1.0]hex-1-yl)-2-oxoessigsäuremethylester in 20 ml THF so zugetropft, daß die Temperatur nicht über -50 °C stieg. Die Lösung wurde anschließend 10 Stunden bei Raumtemperatur gerührt und mit 5 %-iger Schwefelsäure sauer gestellt. Nach Zugabe von 50 ml gesättigter Kochsalzlösung wurde die organische Phase abgetrennt, die wäßrige Phase mit Diethylether extrahiert und die vereinigten organischen Phasen mit gesättigter Kochsalzlösung gewaschen. Zur Stabilisierung wurden 10 mg 2,6-Di-t-butyl-p-kresol (BHT) zugefügt, dann das Lösungsmittel abdestilliert und der Rückstand durch Säulenchromatographie (Pentan/Diethylether 20:1) gereinigt. Es wurden 15,7 g (85 % Ausbeute) an BCHAM als farblose Flüssigkeit erhalten.

IR (film): ν = 3073, 3001, 2953, 2862, 1725 (C=O), 1624 (C=CH₂), 1436, 1366, 1323, 1302, 1216, 1174, 1123, 1018, 996, 941, 856, 812.
¹H-NMR (250 MHz, CDCl₃): δ = 0. 63 (d, ***J*** = 6. 00 Hz, 2 H), 1.12-1.32 (m, 1 H), 1.41-1.47 (m, 1 H), 1.57-1.95 (m, 5 H), 3.73 (s, 3 H), 5.56 (d, ***J*** = 1.65 Hz, 1 H), 6.07 (d, ***J*** = 1.65 Hz, 1 H).
¹³C-NMR (62.9 MHz, CDCl₃, DEPT): δ = 12.9 (-), 21.1 (-), 24.9 (+), 27.5 (-), 30.8 (q), 32.1 (-), 51.5 (+), 124.5 (-), 143.8 (q), 167.4 (q).
MS (70 eV, EI), m/z (%): 166 (37.3) [M⁺], 151 (15.9), 138 (21.4), 134 (58.7), 106 (95.6), 91 (100.0), 79 (62.2), 77 (39.3), 67 (20.6). - C₁₀H₁₄O₂ (166.09938).

### Beispiel 2 :

### 1. Stufe: 2-(Bicyclo[3.1.0]hex-1-yl)acrylsäure

Zu einer gut gerührten Lösung von 13,3 g (80 mmol) BCHAM und 10 mg BHT in 160 ml Aceton und 20 ml Wasser wurden unter einer Stickstoffatmosphäre 40 ml (160 mmol) 4N Lithiumhydroxid zugetropft. Die Mischung wurde bei Raumtemperatur gerührt bis der gesamte Ester verbraucht war (DC-Kontrolle, ca. 24 Stunden). Dann wurde das Lösungsmittel abdestilliert, der Rückstand in etwas Wasser aufgenommen und mit 2 x 50 ml Diethylether gewaschen. Danach wurde mit konz. Salzsäure angesäuert und mit Ether extrahiert. Die vereinigten EtherPhasen wurden mit gesättigter Kochsalzlösung gewaschen und über Natriumsulfat getrocknet. Nach Abziehen des Lösungsmittels erhielt man 10,5 g (86 % Ausbeute) eines weißen Feststoffes.

IR (film): ν = 3436, 3067, 3028, 3005, 2958, 2937, 2861, 2731, 2601, 1694 (C=O), 1621 (C=CH₂), 1426, 1321, 1304, 1253, 1224, 1180, 1134, 1042, 1022, 954, 916, 804.
¹H-NMR (250 MHz, CDCl₃): δ = 0.60-0.70 (m, 2 H), 1.13-1.34 (m, 1 H), 1.43-1.50 (m, 1 H), 1.58-1.97 (m, 5 H), 5.71 (d, ***J*** = 1.62 Hz, 1 H), 6.28 (d, ***J*** = 1.62 Hz, 1 H).
¹³C-NMR (62.9 MHz, CDCl₃, DEPT): δ = 13.0 (_), 21.2 (_), 24.9 (+), 27.5 (_), 30.6 (q), 32.2 (_), 127.3 (_), 143.3 (q), 172.8 (q).
MS (70 eV, EI), m/z (%): 152 (<0.1) [M⁺], 107 (0.7), 78 (100.0), 77 (18.0), 52 (14.1), 51 (12.7). - C₉H₁₂O₂ (152.19): Ber. C 71.03, H 7.95; Gef. C 71.30, H 7.75.

### 2.Stufe: 1,6-Bis-[2-bicyclo[3.1.0]hex-1-ylacroyloxy]hexan (BHAH)

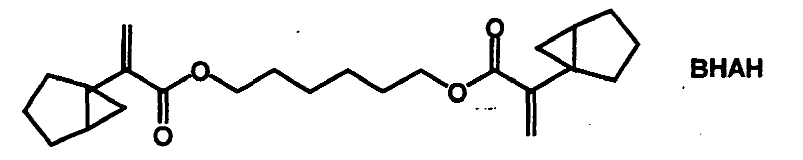

Zu einer Lösung von 11,0 g (72.2 mmol) 2-(Bicyclo[3.1.0]hex-1-yl)acrylsäure, 4,27 g (36,1 mmol) 1,6-Hexandiol und 19,0 g (72,4 mmol) Triphenylphosphin in 145 ml wasserfreiem THF wurden unter Rühren bei -78 °C 12,6 g (72,3 mmol) Azodicarbonsäurediethylester so zugetropft, daß die Temperatur nicht über -70 °C stieg. Die Mischung wurde für 30 min gerührt, dann das Kältebad entfernt und auf Raumtemperatur erwärmen gelassen. Das Lösungsmittel wurde im Vakuum bei Raumtemperatur entfernt und danach der Rückstand in 500 ml Pentan aufgenommen. Die Lösung wurde über Kieselgel filtriert, wobei das Kieselgel mit Pentan/Diethylether (20:1) gewaschen wurde. Das Lösungsmittel wurde erneut im Vakuum bei Raumtemperatur abdestilliert und der verbleibende Rückstand durch Säulenchromatographie (Pentan/Diethylether, 20:1) gereinigt. Es wurden 11,7 g (84 % Ausbeute) von BHAH als farblose Flüssigkeit erhalten.

IR (film): ν = 3067, 3028, 3001, 2953, 2861, 1719 (C=O), 1624 (C=CH₂), 1476, 1451, 1364, 1292, 1213, 1175, 1119, 1017, 987, 941,813.
¹H-NMR (250 MHz, CDCl₃): δ = 0.58-0.66 (m, 4 H), 1.12-1.33 (m, 2 H), 1.39-1.46 (m, 6 H), 1.57-1.74 (m, 8 H), 1.77-1.94 (m, 6 H), 4.13 (t, ***J*** =6.87 Hz, 4 H), 5.56 (d, ***J*** = 1.75 Hz, 2 H), 6.09 (d, *J* = 1.75 Hz, 2 H).
¹³C-NMR (62.9 MHz, CDCl₃, DEPT): δ = 12.9 (-), 21.2 (-), 25.1 (+), 25.7 (-), 27.6 (-), 28.5 (-), 30.8 (q), 32.3 (-), 64.3 (+), 124.6 (-), 144.1 (q), 167.1 (q).
MS (70 eV, EI), m/z (%): 386.3 (14.3) [M⁺], 268 (19.8), 240 (24.6), 152 (38.3), 135 (100.0), 107 (94), 91 (38). - C₂₄H₃₄O₄ (386.24571): 386.2457 (korrekte HRMS).

### Beispiel 3: Radikalische Homopolymerisation von BCHAM in Lösung

In einem Schlenkgefäß wurden zu einer Lösung von BCHAM (2,0 mol/l) in Chlorbenzol 2,0 mol-% (bezogen auf das Monomer) AIBN gegeben. Nach dem Entgasen der Monomerlösung und Verschließen des Schlenkgefäßes unter Argon wurde die Reaktionsmischung im thermostatierten Wasserbad bei 65 °C polymerisiert. Nach 15 h wurde die Polymerisation durch Ausfällen des Polymerisats mit der zehnfachen Menge Methanol abgebrochen. Das gebildete Polymer wurde abfiltriert und bis zur Gewichtskonstanz getrocknet. Die Ausbeute betrug nahezu 100 % an einem weißen Homopolymerisat mit einer zahlenmittleren Molmasse von 120.000 g/mol und einer Glasübergangstemperatur von ca. 90 °C. Die ¹H- und ¹³C-NMR-Spektren des gebildeten Polymers belegen, daß die Polymerisation von BCHAM unter Öffnung des Cyclopropan-Rings verlaufen ist.

### Beispiel 4: Radikalische Homopolymerisation von BHAH in Substanz

Das Monomer BHAH wurde in Substanz mit AIBN (2,5 mol-%) bei 65 °C polymerisiert. Es ergab sich nach 15 h ein transparentes und unlösliches Polymerisat. Die Unlöslichkeit des Produkts zeigt, daß weitgehend beide polymerisationsfähigen Gruppen des Ausgangsmonomeren in die Polymernetzwerkbildung einbezogen waren.

### Beispiel 5: Radikalische Copolymerisation von BCHAM mit MMA

Analog zur Homopolymerisation in Lösung (Beispiel 3) wurde eine Monomermischung aus BCHAM (1,0 mol/l), Methylmethacrylat (MMA, 1,0 mol/l) und AIBN (2,5 mol-%) in Chlorbenzol hergestellt und polymerisiert. Die Ausbeute an Copolymer mit einer zahlenmittleren Molmasse von 84.300 g/mol und einer Glasübergangstemperatur von ca. 85 °C betrug nach 15 Minuten 45 %. Dabei wurde ¹H-NMR-spektroskopisch eine molare Copolymerzusammensetzung von BCHAM:MMA = 1,00:0,63 ermittelt. Dieses Ergebnis belegt die größere Reaktivität des bicyclischen Cyclopropanderivats BCHAM im Vergleich zum Methacrylat MMA.

### Beispiel 6: Radikalische Copolymerisation von BCHAM mit UDMA

Zur Bestimmung des Polymerisationsschrumpfs wurde eine Mischung aus 50 Vol.-% BCHAM und 50 Vol.-% UDMA (dem Urethandimethacrylat aus 2 Mol 2-Hydroxyethylmethacrylat und 1 Mol 2,2,4-Trimethylhexamethylendiisocyanat), hergestellt, mit 0,3 Gew.-%. (bezogen auf die Gesamtmischung) Campherchinon (Photoinitiator) und 0,4 Gew.-% 4-(Dimethylamino)-benzoesäureethylester (Aminbeschleuniger) versetzt und dann mit einer dentalen Lichtquelle (Spectramat, Ivoclar Vivadent AG) bestrahlt. Aus der Differenz der bestimmten Dichten der Monomermischung und des gebildeten Polymerisats wurde unter Berücksichtigung des Polymerisationsschrumpfs von reinem UDMA (Δ_{P}V = 6,1 %) ein Polymerisationsschrumpf von 7,0 % berechnet. Aus den Ergebnissen der Polymerisation einer analogen Mischung von MMA und UDMA (50 :50) wurde der für MMA bekannte Polymerisationsschrumpf von 20,7 % berechnet. Decandioldimethacrylat weist einen Polymerisationsschrumpf von 11 % auf.

Der Vergleich der Beispiele 5 und 6 zeigt, daß sich die erfindungsgemäßen bicyclischen Vinylcyclopropanderivate im Vergleich zu Methacrylaten durch eine höhere Reaktivität und einen geringeren Polymerisationsschrumpf auszeichnen.

### Beispiel 7: Herstellung eines Dentalzements auf der Basis des bicyclischen Vinylcyclopropanderivats aus Beispiel 1

Entsprechend der nachfolgend aufgeführten Tabelle 1 wurde ein Kompositbefestigungszement auf der Basis von (A) einer Methacrylatmischung (Vergleich) und (B) des Monomer BCHAM aus Beispiel 1 mittels eines Walzenstuhles (Typ Exakt, Exakt Apparatebau, Norderstedt) hergestellt. Von den Materialien wurden Prüfkörper mit den Abmessungen 2 x 2 x 20 mm präpariert, die durch zweimaliges Bestrahlen für je 3 Minuten mit einer dentalen Lichtquelle (Spectramat, Ivoclar Vivadent AG) gehärtet wurden. Anschließend wurden die mechanischen Eigenschaften der Prüfkörper nach der ISO-Norm 4049 ermittelt.

Aus Tabelle 2 ist ersichtlich, daß das erfindungsgemäße Material B nach dem Härten in seinen mechanischen Eigenschaften in jeder Hinsicht mit dem Vergleichsmaterial A auf der Basis einer konventionellen Methacrylatmischung vergleichbar ist.

**Tabelle 1**

| **Zusammensetzung der untersuchten Zemente** | | |
|---|---|---|
| **Bestandteil** | **Material A¹⁾** | **Material B** |
| | Anteil (Gew.-%) | Anteil (Gew.-%) |
| Urethandimethacrylat ²⁾ | 31,6 | 31,6 |
| Decandioldimethacrylat | 7,8 | - |
| BCHAM (aus Bsp. 1) | - | 7,8 |
| pyrogene Kieselsäure (Aerosil OX-50, Degussa) | 41,2 | 41,2 |
| Ytterbiumtrifluorid (Rhone-Poulenc) | 18,7 | 18,7 |
| Photoinitiator³⁾ | 0, 7 | 0, 7 |

| | | |
|---|---|---|
| ¹⁾ Vergleichsbeispiel ²⁾ Urethandimethacrylat aus 2 mol 2-Hydroxyethylmethacrylat und 1 mol 2,2,4-Trimethylhexamethylendiisocyanat-1,6 ³⁾ 1:1-Mischung aus Campherchinon und N,N-Diethyl-3,5-di-tert.-butylanilin | | |

**Tabelle 2**

| **Mechanische Eigenschaften der untersuchten Zemente** | | |
|---|---|---|
| **Materialeigenschaft** | **Material A¹⁾** | **Material B** |
| Biegefestigkeit (MPa) nach 24 h | 95 | 104 |
| Biegefestigkeit (MPa) nach 24 h WL²⁾ | 101 | 106 |
| Biegefestigkeit (MPa) nach 7 d WL | 111 | 119 |
| Biege-E-Modul (Gpa) nach 24 h | 4,71 | 4,78 |
| Biege-E-Modul (Gpa) nach 24 h WL | 4,90 | 4,69 |
| Biege-E-Modul (Gpa) nach 7 d WL | 5,13 | 5,14 |

| | | |
|---|---|---|
| ¹⁾ Vergleichsbeispiel ²⁾ WL = Wasserlagerung der Prüfkörper | | |

Das Beispiel zeigt, daß Dentalmaterialien auf der Basis erfindungsgemäßer bicyclischer Cyclopropanderivate wie BCHAM trotz verbesserter Reaktivität und verringertem Polymerisationsschrumpf keine Nachteile im Hinblick auf die mechanischen Eigenschaften aufweisen.

## Patentansprüche

1. Zusammensetzung, **dadurch gekennzeichnet, daß** sie ein bicyclisches Cyclopropanderivat der allgemeinen Formel (I) 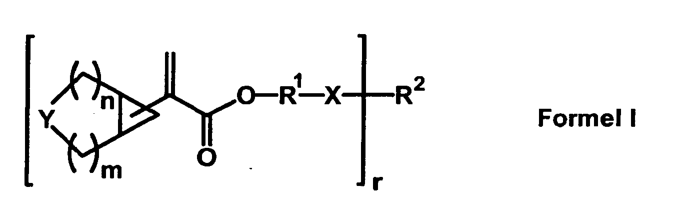 in der R¹, R², X, Y, n, m und r unabhängig voneinander die folgenden Bedeutungen haben:
n+m = 0 bis 8;
r = 1 bis 4;
R¹ = entfällt oder ein C₁-C₂₀-Alkylen-Rest, der durch O oder S unterbrochen sein kann, ein cycloaliphatischer C₄-C₁₂-Rest, ein bicyclischer C₄-C₁₂-Rest, ein C₆-C₁₄-Arylen- oder C₇-C₂₀-Alkylenarylen-Rest;
R² ist für r = 1: ein C₁-C₂₀-Alkyl-Rest, der durch O oder S unterbrochen sein kann, ein cycloaliphatischer C₄-C₁₂-Rest, ein bicyclischer C₄-C₁₂-Rest, ein C₆-C₁₄-Aryl- oder C₇-C₂₀-Alkylaryl-Rest;
für r > 1: ein r-fach substituierter aliphatischer C₁- bis C₂₀-Rest, der durch O oder S unterbrochen sein kann, ein cycloaliphatischer C₄-C₁₂-Rest, ein aromatischer C₆-C₁₄-Rest oder aliphatisch-aromatischer C₇-C₂₀-Rest;
X = entfällt, -CO-O-, -CO-NH- oder -O-CO-NH- und
Y = CH₂, O oder S
und einen Initiator für die radikalische Polymerisation enthält.

2. Zusammensetzung nach Anspruch 1, **dadurch gekennzeichnet, daß** mindestens eine Variable der Formel (I) eine der folgenden Bedeutungen hat:
n+m = 1 bis 5;
r = 1 bis 3;
R¹ = entfällt oder ein C₁-C₁₀-Alkylen-Rest, der durch O unterbrochen sein kann, Cyclohexylen, ein bicyclischer C₆-C₉-Rest, Phenylen oder ein C₇-C₁₀-Alkylenarylen-Rest;
R² ist für r = 1: ein C₁-C₆-Alkyl-Rest, der durch O unterbrochen sein kann, ein cycloaliphatischer oder bicyclischer C₆-C₈-Rest, ein C₆-C₁₀-Aryl- oder C₇-C₁₀-AlkylarylRest;
für r > 1: ein r-fach substituierter aliphatischer C₁- bis C₁₂-Rest, der durch O unterbrochen sein kann, ein cycloaliphatischer C₅-C₇-Rest, ein aromatischer C₆-C₁₀-Rest oder aliphatisch-aromatischer C₇-C₁₀-Rest;
X = entfällt, -CO-O- oder -O-CO-NH- und
Y = CH₂ oder O.

3. Zusammensetzung nach Anspruch 1 oder 2, **dadurch gekennzeichnet, daß** mindestens eine Variable der Formel (I) eine der folgenden Bedeutungen hat:
n+m = 2 oder 3;
r = 1 oder 2;
R¹ = entfällt, eine -(CH₂)₁₋₄- Rest, der durch ein O unterbrochen sein kann, Cyclohexylen oder Phenylen;
R² ist für r = 1: ein C₁-C₄-Alkyl-Rest, der durch ein O unterbrochen sein kann, Cyclohexyl, Bicyclo[2.2.1]heptyl oder;
für r > 1: ein r-fach substituierter aliphatischer C₂- bis C₆-Rest, ein r-wertiger Cyclohexan-Rest oder ein r-wertiger Benzol-Rest;
X = entfällt oder -CO-O- und
Y = CH₂.

4. Zusammensetzung nach einem der Ansprüche 1 bis 3, **dadurch gekennzeichnet, daß** r gleich 1 ist und R² unsubstituiert oder durch Alkyl, Halogen, OCH₃, OC₂H₅, Vinyl, Propenyl, (Meth) acryl, COOR³, SiCl₃, Si(OR⁴)₃, oder eine mesogene Gruppe substituiert ist, wobei R³ = H, ein C₁- bis C₁₀-Alkyl- oder ein Phenyl-Rest und R⁴ = H oder ein C₁- bis C₁₀-Alkyl-Rest ist.

5. Zusammensetzung nach einem der Ansprüche 1 - 3, **dadurch gekennzeichnet, daß** r größer als 1 ist und R² unsubstituiert oder durch Alkyl, Halogen, OCH₃, OC₂H₅, Vinyl, Propenyl, (Meth)acryl, CO-OR³ oder eine mesogene Gruppe substituiert ist, wobei R³ = H oder C₁- bis C₁₀-Alkyl- oder ein Phenyl-Rest ist.

6. Zusammensetzung nach einem der Ansprüche 1 bis 5, **dadurch gekennzeichnet, daß** sie zusätzlich ein radikalisch polymerisierbares Monomer enthält.

7. Zusammensetzung nach einem der Ansprüche 1 bis 6, **dadurch gekennzeichnet, daß** sie ein monofunktionelles und/oder ein mehrfunktionelles radikalisch polymerisierbares Monomer enthält.

8. Zusammensetzung nach Anspruch 7, **dadurch gekennzeichnet, daß** sie als monofunktionelles radikalisch polymerisierbares Monomer ein Urethan aus 2-(Hydroxymethyl)acrylsäureethylester und einem Diisocyant, wie 2,2,4-Trimethylhexamethylendiisocyanat oder Isophorondiisocyanat, ein vernetzendes Pyrrolidon, wie 1,6-Bis(3-vinyl-2-pyrrolidonyl)-hexan, ein Bisacrylamid, wie Methylen- oder Ethylenbisacrylamid, ein Bis(meth)-acrylamid, wie N,N'-Diethyl-1,3-bis(acrylamido)-propan, 1,3-Bis(methacrylamido)-propan, 1,4-Bis(acrylamido)-butan oder N,N'-Bis-(acryloyl)-piperazin, oder eine Mischung von zwei oder mehr dieser Monomere enthält.

9. Zusammensetzung nach Anspruch 7 oder 8, **dadurch gekennzeichnet, daß** sie als mehrfunktionelles radikalisch polymerisierbares Monomer ein bi- oder mehrfunktionelles Acrylat oder Methacrylat, wie Bisphenol-A-di(meth)acrylat, Bis-GMA (ein Additionsprodukt aus Methacrylsäure und Bisphenol-A-diglycidylether), UDMA (ein Additionsprodukt aus Hydroxyethylmethacrylat und 2,2,4-Trimethylhexamethylendiisocyanat), Di-, Tri- oder Tetraethylenglycoldi(meth)acrylat, Decandioldi(meth)acrylat, Trimethylolpropantri(meth)acrylat, Pentaerythrittetra(meth)acrylat, Butandioldi(meth)acrylat, 1,10-Decandioldi(meth)acrylat, 1,12-Dodecandioldi(meth)acrylat oder eine Mischung aus zwei oder mehr dieser Monomere enthält.

10. Zusammensetzung nach einem der Ansprüche 1 bis 9, **dadurch gekennzeichnet, daß** sie zusätzlich Füllstoff enthält.

11. Zusammensetzung nach einem der Ansprüche 6 bis 10, **dadurch gekennzeichnet, daß** sie
a) 1 bis 95 Gew.-% bicyclisches Cyclopropanderivat gemäß einem der Ansprüche 1 bis 5;
b) 0,01 bis 5 Gew.-% Initiator für die radikalische Polymerisation; und
c) 0 bis 94 Gew.-% radikalisch polymerisierbares Monomer;
enthält.

12. Zusammensetzung nach Anspruch 11, **dadurch gekennzeichnet, daß** sie
a) 1 bis 80 Gew.-% bicyclisches Cyclopropanderivat gemäß einem der Ansprüche 1 bis 5;
b) 0,01 bis 5 Gew.-% Initiator für die radikalische Polymerisation
c) 0 bis 60 Gew.-% radikalisch polymerisierbares Monomer;
d) 0 bis 20 Gew.-% Füllstoff;
und/oder
e) 0 bis 40 Gew.-% Lösungsmittel enthält.

13. Zusammensetzung nach Anspruch 11, **dadurch gekennzeichnet, daß** sie
a) 1 bis 60 Gew.-% bicyclisches Cyclopropanderivat gemäß einem der Ansprüche 1 bis 5;
b) 0,01 bis 5 Gew.-% Initiator für die radikalische Polymerisation
c) 0 bis 60 Gew.-% radikalisch polymerisierbares Monomer;
und/oder
d) 20 bis 60 Gew.-% Füllstoff enthält.

14. Zusammensetzung nach Anspruch 11, **dadurch gekennzeichnet, daß** sie
a) 1 bis 45 Gew.-% bicyclisches Cyclopropanderivat gemäß einem der Ansprüche 1 bis 5;
b) 0,01 bis 5 Gew.-% Initiator für die radikalische Polymerisation
c) 0 bis 50 Gew.-% radikalisch polymerisierbares Monomer;
und/oder
d) 30 bis 85 Gew.-% Füllstoff enthält.

15. Zusammensetzung nach Anspruch 11, **dadurch gekennzeichnet, daß** sie
a) 1 bis 95 Gew.-% bicyclisches Cyclopropanderivat gemäß einem der Ansprüche 1 bis 5;
b) 0,01 bis 5 Gew.-% Initiator für die radikalische Polymerisation
c) 0 bis 60 Gew.-% radikalisch polymerisierbares Monomer;
und/oder
d) 0 bis 20 Gew.-% Füllstoff enthält.

16. Verwendung eines bicyclischen Cyclopropanderivats der allgemeinen Formel (I) 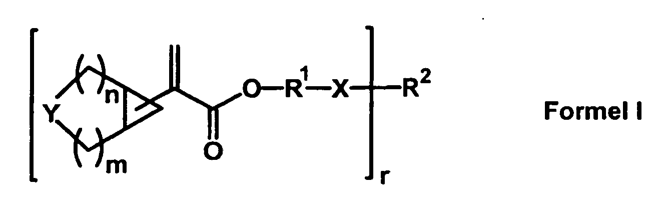 in der R¹, R², X, Y, n, m und r unabhängig voneinander die folgenden Bedeutungen haben:
n+m = 0 bis 8;
r = 1 bis 4;
R¹ = entfällt oder ein C₁-C₂₀-Alkylen-Rest, der durch oder S unterbrochen sein kann, ein cycloaliphatischer C₄-C₁₂-Rest, ein bicyclischer C₄-C₁₂-Rest, ein C₆-C₁₄-Arylen- oder C₇-C₂₀-Alkylenarylen-Rest;
R² ist für r = 1: ein C₁-C₂₀-Alkyl-Rest, der durch oder S unterbrochen sein kann, ein cycloaliphatischer C₄-C₁₂-Rest, ein bicyclischer C₄-C₁₂-Rest, ein C₆-C₁₄-Aryl- oder C₇-C₂₀-Alkylaryl-Rest;
für r > 1: ein r-fach substituierter aliphatischer C₁- bis C₂₀-Rest, der durch O oder S unterbrochen sein kann, ein cycloaliphatischer C₄-C₁₂-Rest, ein aromatischer C₆-C₁₄-Rest oder aliphatisch-aromatischer C₇-C₂₀-Rest;
X = entfällt, -CO-O-, -CO-NH- oder -O-CO-NH- und
Y = CH₂, O oder S
zur Herstellung eines Dentalmaterials.

17. Verwendung einer Zusammensetzung gemäß einem der Ansprüche 1 bis 15 als Dentalmaterial

18. Verwendung einer Zusammensetzung nach Anspruch 12 als Adhäsiv.

19. Verwendung einer Zusammensetzung nach Anspruch 13 als Zement.

20. Verwendung einer Zusammensetzung nach Anspruch 14 als Füllmaterial.

21. Verwendung einer Zusammensetzung nach Anspruch 15 als Beschichtungsmaterial.

## Claims

1. Composition, **characterised in that** it comprises a bicyclic cyclopropane derivative of the general Formula (I) 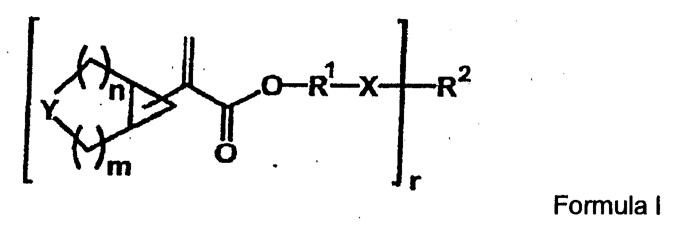 in which R¹, R², X, Y, n, m and r, independently of one another, have the following meanings:
n+m = 0 to 8;
r = 1 to 4; 4;
R¹ = is absent or a C₁-C₂₀ alkylene group that can be interrupted by O or S, a cycloaliphatic C₄-C₁₂ group, a bicyclic C₄-C₁₂ group, a C₆-C₁₄ arylene or C₇-C₂₀ alkylenearylene group;
R² is for r = 1: a C₁-C₂₀ alkyl group that can be interrupted by O or S, a cycloaliphatic C₄-C₁₂ group, a bicyclic C₄-C₁₂ group, a C₆-C₁₄ aryl or C₇-C₂₀ alkylaryl group;
for r > 1: an r-times substituted aliphatic C₁-C₂₀ group that can be interrupted by O or S, a cycloaliphatic C₄-C₁₂ group, an aromatic C₆-C₁₄ group or aliphatic-aromatic C₇-C₂₀ group;
X = is absent, -CO-O-, -CO-NH- or -O-CO-NH- and
Y = CH₂, O or S
and an initiator for radical polymerisation.

2. Composition according to claim 1, **characterised in that** at least one variable of the Formula (I) has one of the following meanings:
n + m - 1 to 5;
r = 1 to 3;
R¹ = is absent, or a C₁-C₁₀ alkylene group that can be interrupted by O, cyclohexylene, a bicyclic C₆-C₉ group, phenylene or a C₇-C₁₀ alkylenearylene group;
R² is for r = 1: a C₁-C₆ alkyl group which can be interrupted by O, a cycloaliphatic or bicyclic C₆-C₈ group, a C₆-C₁₀ aryl or C₇-C₁₀ alkylaryl group;
for r > 1: an r-times substituted aliphatic C₁ to C₁ group that can be interrupted by O, a cycloaliphaticC₅-C₇ group, an aromatic C₆-C₁₀ group or aliphatic-aromatic C₇-C₁₀ group;
X = is absent, -CO-O- or -O-CO-NH- and
Y = CH₂ or O.

3. Composition according to claim 1 or 2, **characterised in that** at least one variable of the Formula (I) has one of the following meanings:
n + m = 2 or 3;
r = 1 or 2;
R¹ = is absent, a -(CH₂)₁₋₄ group that can be interrupted by O, cyclohexylene or phenylene;
R² is for r = 1: a C₁-C₄ alkyl group that can be interrupted by an O, cyclohexyl, bicyclo [2.2.1] heptyl or;
for r > 1: an r-times substituted aliphatic C₂ to C₆ group, an r-valent cyclohexane group or an r-valent benzene group;
X = is absent or -CO-O- and
Y = CH₂-

4. Composition according to one of claims 1 to 3, **characterised in that** r is equal to 1 and R² is unsubstituted or substituted by alkyl, halogen, OCH₃, OC₂H₅, vinyl, propenyl, (meth)acryl, COOR³, SiCl₃, Si (OR⁴)₃, or a mesogenic group, wherein R³ = H, a C₁ to C₁₀ alkyl or a phenyl group and R⁴ = H or a C₁ to C₁₀ alkyl group.

5. Composition according to one of claims 1 to 3, **characterised in that**, r is greater than 1 and R² is unsubstituted or substituted by alkyl, halogen, OCH₃, OC₂H₅, vinyl, propenyl, (meth)acryl, CO-OR³ or a mesogenic group, wherein R³ = H or C₁ to C₁₀ alkyl or a phenyl group.

6. Composition according to one of claims 1 to 5, **characterised in that** it additionally comprises a radically polymerisable monomer.

7. Composition according to one of claims 1 to 6, **characterised in that** it comprises a monofunctional and/or a polyfunctional radically polymerisable monomer.

8. Composition according to claim 7, **characterised in that** it comprises, as the monofunctional radically polymerisable monomer, a urethane from 2-(hydroxymethyl) acrylic acid ethyl ester and a diisocyanate such as 2,2,4-trimethylhexamethylene diisocyanate or isophorone diisocyanate, a crosslinking pyrrolidone such as 1,6-bis(3-vinyl-2-pyrrolidonyl)-hexane, a bisacrylamide such as methylene or ethylene bisacrylamide, a bis(meth)acrylamide such as N,N'-diethyl-1,3-bis (acrylamido) -propane, 1,3-bis(methacrylamido)-propane, 1,4-bis(acrylamido)-butane or N,N'-bis-(acryloyl)-piperazine, or a mixture of two or more of these monomers.

9. Composition according to claim 7 or 8, **characterised in that** it contains, as the polyfunctional radically polymerisable monomer, a bi- or polyfunctional acrylate or methacrylate such as Bisphenol-A-di(meth)acrylate, bis-GMA (an addition product of methacrylic acid and Bisphenol-A-diglycidyl ether), UDMA (an addition product of hydroxyethyl methacrylate and 2,2,4-trimethylhexamethylene diisocyanate), di-, tri- or tetraethylene glycol di(meth)acrylate, decanediol di(meth)acrylate, trimethylolpropane tri(meth)acrylate, pentaerythritol tetra(meth)acrylate, butanediol di(meth)acrylate, 1,10-decanediol di(meth)acrylate, 1,12-dodecandiol di(meth)acrylate or a mixture of two or more of these monomers.

10. Composition according to one of claims 1 to 9, **characterised in that** it additionally comprises filler.

11. Composition according to one of claims 6 to 10, **characterised in that** it comprises
a) 1 to 95 wt.-% bicyclic cyclopropane derivative according to one of claims 1 to 5;
b) 0.01 to 5 wt.-% initiator for radical polymerisation; and
c) 0 to 94 wt.-% radically polymerisable monomer.

12. Composition according to claim 11, **characterised in that** it contains
a) 1 to 80 wt.-% bicyclic cyclopropane derivative according to one of claims 1 to 5;
b) 0.01 to 5 wt.-% initiator for radical polymerisation
c) 0 to 60 wt.-% radically polymerisable monomer;
d) 0 to 20 wt.-% filler;
and/or
e) 0 to 40 wt.-% solvent.

13. Composition according to claim 11, **characterised in that** it contains
a) 1 to 60 wt.-% bicyclic cyclopropane derivative according to one of claims 1 to 5;
b) 0.01 to 5 wt.-% initiator for radical polymerisation
c) 0 to 60 wt.-% radically polymerisable monomer;
and/or
d) 20 to 60 wt.-% filler.

14. Composition according to claim 11, **characterised in that** it contains
a) 1 to 45 wt.-% bicyclic cyclopropane derivative according to one of claims 1 to 5;
b) 0.01 to 5 wt.-% initiator for radical polymerisation
c) 0 to 50 wt.-% radically polymerisable monomer;
and/or
d) 30 to 85 wt.-% filler.

15. Composition according to claim 11, **characterised in that** it contains
a) 1 to 95 wt.-% bicyclic cyclopropane derivative according to one of claims 1 to 5;
b) 0.01 to 5 wt.-% initiator for radical polymerisation
c) 0 to 60 wt.-% radically polymerisable monomer;
and/or
d) 0 to 20 wt.-% filler.

16. Use of a bicyclic cyclopropane derivative of the general formula (I) for the preparation of a dental material. 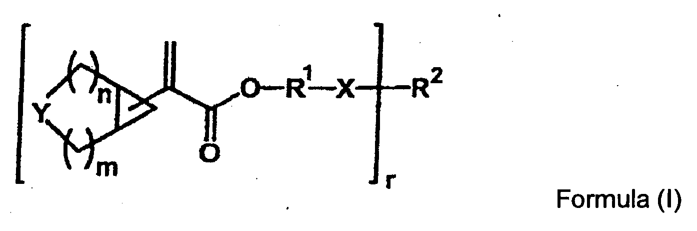 in which R¹, R², X, Y, n, m and r, independently of one another, have the following meanings:
n+m = 0 to 8;
r = 1 to 4;
R¹ = is absent or a C₁-C₂₀ alkylene group that can be interrupted by O or S, a cycloaliphatic C₄-C₁₂ group, a bicyclic C₄-C₁₂ group, a C₆-C₁₄ arylene or C₇-C₂₀ alkylene-arylene group;
R² is for r = 1: a C₁-C₂₀ alkyl group that can be interrupted by O or S, a cycloaliphatic C₄-C₁₂ group, a bicyclic C₄-C₁₂ group, a C₆-C₁₄ aryl or C₇-C₂₀ alkylaryl group;
for r > 1: an r-times substituted aliphatic C₁-C₂₀ group that can be interrupted by O or S, a cycloaliphatic C₄-C₁₂ group, an aromatic C₆-C₁₄ group or aliphatic-aromatic C₇-C₂₀ group;
X = is absent, -CO-O-, -CO-NH- or -O-CO-NH- and
Y = CH₂, O or S.

17. Use of a composition according to one of claims 1 to 15 as dental material.

18. Use of a composition according to claim 12 as adhesive.

19. Use of a composition according to claim 13 as cement.

20. Use of a composition according to claim 14 as filling material.

21. Use of a composition according to claim 15 as coating material.

## Revendications

1. Composition, **caractérisée en ce qu'**elle contient un dérivé bicyclique de cyclopropane de formule générale (I) 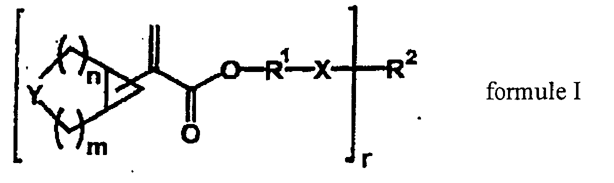 dans laquelle R¹, R², X, Y, n, m et r ont, indépendamment les uns des autres, les significations suivantes :
n+m = 0 à 8 ;
r = 1 à 4 ;
R¹ = est absent ou représente un radical alkylène en C₁-C₂₀ qui peut être interrompu par O ou S, un radical cycloaliphatique en C₄-C₁₂, un radical bicyclique en C₄-C₁₂, un radical arylène en C₆-C₁₄ ou alkylène-arylène en C₇-C₂₀ ;
R² = représente, lorsque r = 1 : un radical alkyle en C₁-C₂₀ qui peut être interrompu par O ou S, un radical cycloaliphatique en C₄-C₁₂, un radical bicyclique en C₄-C₁₂, un radical aryle en C₆-C₁₄ ou alkylaryle en C₇-C₂₀ ;
lorsque r > 1 : un radical aliphatique en C₁-C₂₀ r-fois substitué, qui peut être interrompu par O ou S, un radical cycloaliphatique en C₄-C₁₂, un radical aromatique en C₆-C₁₄ ou un radical aliphatique-aromatique en C₇-C₂₀ ;
X = est absent ou représente -CO-O-, -CO-NH- ou -O-CO-NH- et
Y = représente CH₂, O ou S
et un amorceur pour la polymérisation radicalaire.

2. Composition selon la revendication 1, **caractérisée en ce qu'**au moins l'une des variables de la formule (I) a l'une des significations suivantes :
n+m = 1 à 5 ;
r = 1 à 3 ;
R¹ = est absent ou représente un radical alkylène en C₁-C₁₀ qui peut être interrompu par O, un cyclohexylène, un radical bicyclique en C₆-C₉, un radical phénylène ou un radical alkylène-arylène en C₇-C₁₀;
R² représente, lorsque r = 1 : un radical alkyle en C₁-C₆ qui peut être interrompu par O, un radical cycloaliphatique ou bicyclique en C₆-C₈, un radical aryle en C₆-C₁₀ ou alkylaryle en C₇-C₁₀ ;
lorsque r > 1 : un radical aliphatique en C₁-C₁₂ r-fois substitué, qui peut être interrompu par O, un radical cycloaliphatique en C₅-C₇, un radical aromatique en C₆-C₁₀ ou un radical aliphatique-aromatique en C₇-C₁₀ ;
X = est absent ou représente -CO-O- ou -O-CO-NH- et
Y = représente CH₂ ou O.

3. Composition selon la revendication 1 ou 2, **caractérisée en ce qu'**au moins l'une des variables de la formule (I) a l'une des significations suivantes :
n+m = 2 ou 3 ;
r = 1 ou 2 ;
R¹ = est absent ou représente un radical -(CH₂)₁₋₄- qui peut être interrompu par un O, cyclohexylène ou phénylène ;
R² représente, lorsque r = 1 : un radical alkyle en C₁-C₄ qui peut être interrompu par O, cyclohexyle, bicyclo[2.2.1]heptyle ; ou
lorsque r > 1 : un radical aliphatique en C₂-C₆ r-fois substitué, un radical cyclohexane r-valent ou un radical benzénique r-valent ;
X est absent ou représente -CO-O- et
Y représente CH₂.

4. Composition selon l'une quelconque des revendications 1 à 3, **caractérisée en ce que** r est égal à 1 et R² est non substitué ou substitué par un groupe alkyle, halogène, OCH₃, OC₂H₅, vinyle, propényle, (méth)acrylique, COOR³, SiCl₃, Si(OR⁴)₃ ou un groupe mésogène, R³ représentant H, un radical alkyle en C₁-C₁₀ ou un radical phényle et R⁴ représentant H ou un radical alkyle en C₁-C₁₀.

5. Composition selon l'une quelconque des revendications 1 à 3, **caractérisée en ce que** r est supérieure à 1 et R² est non substitué ou substitué par un groupe alkyle, halogène, OCH₃, OC₂H₅, vinyle, propényle, (méth)acrylique, CO-OR³, ou un groupe mésogène, R³ représentant H ou un radical alkyle en C₁-C₁₀ ou un radical phényle.

6. Composition selon l'une quelconque des revendications 1 à 5, **caractérisée en ce qu'**elle contient en outre un monomère apte à la polymérisation radicalaire.

7. Composition selon l'une quelconque des revendications 1 à 6, **caractérisée en ce qu'**elle contient un monomère monofonctionnel et/ou un monomère polyfonctionnel, apte à la polymérisation radicalaire.

8. Composition selon la revendication 7, **caractérisée en ce qu'**elle contient, en tant que monomère monofonctionnel apte à la polymérisation radicalaire, un uréthane à base de 2-(hydroxyméthyl)acryloate d'éthyle et d'un diisocyanate tel que le 2,2,4-triméthylhexaméthylène-diisocyanate ou l'isophorone-diisocyanate, une pyrrolidone réticulable telle que le 1,6-bis(3-vinyl-2-pyrrolidonyl)hexane, un bisacrylamide tel que le méthylène-bisacrylamide ou l'éthylène-bis-acrylamide, un bis(méth)acrylamide tel que le N,N'-diéthyl-1,3-bis(acrylamido)propane, le 1,3-bis(méthacrylamido)propane, le 1,4-bis(acrylamido)butane ou la N,N'-bis(acryloyl)-pipérazine ou un mélange de ces deux ou plus de ces monomères.

9. Composition selon la revendication 7 ou 8, **caractérisée en ce qu'**elle contient, en tant que monomère polyfonctionnel apte à la polymérisation radicalaire, un acrylate ou méthacrylate bi- ou polyfonctionnel tel que le di(méth)acrylate de bisphénol A, le bis-GMA (un produit d'addition d'acide méthacrylique et d'éther diglycidylique de bisphénol A), l'UDMA (un produit d'addition de méthacrylate d'hydroxyéthyle et 2,2,4-triméthylhexaméthylène-diisocyanate), le di(méth)acrylate de di-, tri- ou tétraéthylèneglycol, le di(méth)acrylate de décanediol, le tri(méth)acrylate de triméthylolpropane, le tétra(méth)acrylate de pentaérythritol, le di(méth)acrylate de butanediol, le di(méth)acrylate de 1,10-décanediol, le di(méth)acrylate de 1,12-dodécanediol ou un mélange de ces deux ou plus de ces monomères.

10. Composition selon l'une quelconque des revendications 1 à 9, **caractérisée en ce qu'**elle contient en outre une charge.

11. Composition selon l'une quelconque des revendications 6 à 10, **caractérisée en ce qu'**elle contient
(a) 1 à 95 % en poids d'un dérivé bicyclique de cyclopropane selon l'une quelconque des revendications 1 à 5,
(b) 0,01 à 5 % en poids d'amorceur pour la polymérisation radicalaire ; et
(c) 0 à 94 % en poids d'un monomère apte à la polymérisation radicalaire.

12. Composition selon la revendication 11, **caractérisée en ce qu'**elle contient
(a) 1 à 80 % en poids d'un dérivé bicyclique de cyclopropane selon l'une quelconque des revendications 1 à 5 ,
(b) 0,01 à 5 % en poids d'amorceur pour la polymérisation radicalaire,
(c) 0 à 60 % en poids d'un monomère apte à la polymérisation radicalaire,
(d) 0 à 20 % en poids de charge et/ou
(e) 0 à 40 % en poids de solvant.

13. Composition selon la revendication 11, **caractérisée en ce qu'**elle contient
(a) 1 à 60 % en poids d'un dérivé bicyclique de cyclopropane selon l'une quelconque des revendications 1 à 5 ,
(b) 0,01 à 5 % en poids d'amorceur pour la polymérisation radicalaire,
(c) 0 à 60 % en poids d'un monomère apte à la polymérisation radicalaire et/ou
(d) 20 à 60 % en poids de charge.

14. Composition selon la revendication 11, **caractérisée en ce qu'**elle contient
(a) 1 à 45 % en poids d'un dérivé bicyclique de cyclopropane selon l'une quelconque des revendications 1 à 5,
(b) 0,01 à 5 % en poids d'amorceur pour la polymérisation radicalaire,
(c) 0 à 50 % en poids d'un monomère apte à la polymérisation radicalaire et/ou
(d) 30 à 85 % en poids de charge.

15. Composition selon la revendication 11, **caractérisée en ce qu'**elle contient
(a) 1 à 95 % en poids d'un dérivé bicyclique de cyclopropane selon l'une quelconque des revendications 1 à 5,
(b) 0,01 à 5 % en poids d'amorceur pour la polymérisation radicalaire,
(c) 0 à 60 % en poids d'un monomère apte à la polymérisation radicalaire et/ou
(d) 0 à 20 % en poids de charge.

16. Utilisation d'un dérivé bicyclique de cyclopropane de formule générale (I) 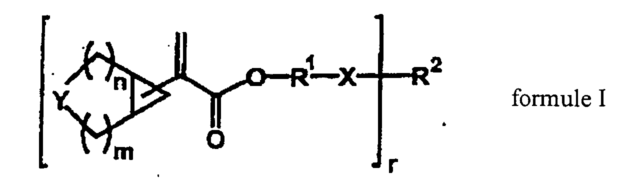 dans laquelle R¹, R², X, Y, n, m et r ont, indépendamment les uns des autres, les significations suivantes :
n+m = 0 à 8 ;
r = 1 à 4;
R¹ = est absent ou représente un radical alkylène en C₁-C₂₀ qui peut être interrompu par O ou S, un radical cycloaliphatique en C₄-C₁₂, un radical bicyclique en C₄-C₁₂, un radical arylène en C₆-C₁₄ ou alkylène-arylène en C₇-C₂₀;
R² représente, lorsque r = 1 : un radical alkyle en C₁-C₂₀ qui peut être interrompu par O ou S, un radical cycloaliphatique en C₄-C₁₂, un radical bicyclique en C₄-C₁₂, un radical aryle en C₆-C₁₄ ou alkylaryle en C₇-C₂₀ ;
lorsque r > 1 : un radical aliphatique en C₁-C₂₀ r-fois substitué, qui peut être interrompu par O ou S, un radical cycloaliphatique en C₄-C₁₂, un radical aromatique en C₆-C₁₄ ou un radical aliphatique-aromatique en C₇-C₂₀;
X = est absent ou représente -CO-O-, -CO-NH- ou -O-CO-NH- et
Y = représente CH₂, O ou S
pour la fabrication d'un matériau dentaire.

17. Utilisation d'une composition selon l'une quelconque des revendications 1 à 15, en tant que matériau dentaire.

18. Utilisation d'une composition selon la revendication 12, en tant qu'adhésif.

19. Utilisation d'une composition selon la revendication 13, en tant que colle.

20. Utilisation d'une composition selon la revendication 14, en tant que matériau d'obturation.

21. Utilisation d'une composition selon la revendication 15, en tant que matériau de revêtement.
